# EUROPEAN PATENT SPECIFICATION

(11) **EP 4 605 051 B1**
(45) Date of publication and mention of the grant of the patent: **25.11.2026**
(21) Application number: 23800930.2
(22) Date of filing: 20.10.2023
(51) Int. Cl.: A61M 21/00, G16H 20/00, G16H 50/30, G16H 50/20, G16H 20/30, G16H 20/70, G16H 40/63

(54) **A METHOD OF CLOSED-LOOP MODULATION OF AUDIO DATA FOR NEURAL OSCILLATION SUPPRESSION OR ENHANCEMENT**
VERFAHREN ZUR MODULATION VON AUDIODATEN ZUR UNTERDRÜCKUNG ODER VERBESSERUNG VON NEURONALER SCHWINGUNG
PROCÉDÉ DE MODULATION EN BOUCLE FERMÉE DE DONNÉES AUDIO POUR LA SUPPRESSION OU L'AMÉLIORATION D'OSCILLATION NEURONALE

(30) Priority: 21.10.2022 GB 202215652
(43) Date of publication of application: 27.08.2025
(73) Proprietor: Rickert, Joern, 67435 Neustadt / Weinstraße (DE); Jackson, Andrew, Newcastle NE2 1HP (GB); Baker, Stuart, Newcastle NE3 1UL (GB)
(72) Inventor: Rickert, Joern, 67435 Neustadt / Weinstraße (DE); Jackson, Andrew, Newcastle NE2 1HP (GB); Baker, Stuart, Newcastle NE3 1UL (GB)
(74) Representative: 2s-ip Schramm Schneider Bertagnoll Patent- und Rechtsanwälte Part mbB
(86) International application number: PCT/GB2023/052751
(87) International publication number: WO 2024/084244

(56) References cited:
- WO-A1-2021/016624
- CN-A- 111 345 784
- US-A- 4 883 067
- US-A1- 2019 255 350
- US-A1- 2021 161 418
- US-A1- 2021 338 973

## Description

The present invention relates to a method and system for closed-loop modulation of audio data to selectively enhance or supress brain oscillation activity in a desired frequency range in a desired region of a user's brain. In particular, but not exclusively, the present invention relates to a system and methodology whereby a human user receives external auditory stimulation based on brain activity to increase or decrease oscillatory activity in different frequency bands in one or more different brain areas.

Altering brain oscillations may have therapeutic or non-therapeutic effects such as improving memory, improving attention, and/or reducing a perception of anxiety or stress or fatigue or the like.

Increasingly, neuroscience is linking brain oscillations at different frequencies to a range of normal and pathological mental states, for example alpha activity around 10 Hz linked to attention, theta activity around 5 Hz linked to memory, delta activity below 4 Hz linked to sleep, and the like. These brain oscillations are targets for neuromodulation therapies that aim to alter brain function by modulating activity at different frequencies. Applications include non-medical 'enhancement', for example facilitating attentiveness, sleep or memory in healthy people, to clinical applications including dementia, mood disorders, epilepsy, mental health and many more.

Neuroscience has thus to date identified neural correlates of many normal and pathological brain states. In particular, these are often characterised by too much or too little oscillatory activity in different frequency bands (alpha, beta, theta, etc.) in different brain areas. Research suggests that altering brain oscillations using a variety of methods may have numerous beneficial effects, such as improving memory, relaxation, attention, sleep as well as improving the symptoms of many neurological conditions, e.g. ADHD, depression, anxiety, insomnia.

One existing technology for influencing brain oscillations is neurofeedback. In a typical implementation, a brain oscillation is measured and used to control sensory feedback. The user learns to control this signal and thereby boost or suppress the amplitude of neural oscillation.

Another approach is stimulation, which may be delivered either directly to the brain through electrical/magnetic/ultrasound stimulation, or through sensory pathways.

Most neuromodulation is delivered as electromagnetic stimulation, either using implanted electrodes, such as Deep Brain Stimulation, or non-invasive methods, such as Transcranial Magnetic Stimulation. The CANDO project (www.cando.ac.uk) is developing a brain implant to deliver optogenetic therapy to apply this principle to the control of epileptic seizures, further details available as Zaaimi B et al. Nat Biomed Eng. 2023 7(4): 559-575 available at https://www.researchsquare.com/article/rs-78230/v1 or https://www.ncbi.nlm.nih.gov/pmc/articles/PMC7614485/.

The applications for invasive neuromodulation techniques such as the CANDO device are limited by the need for surgery. Some non-invasive methods are limited in terms of regions of the brain that can be stimulated and the need for bulky equipment that limits the duration that therapy can be delivered.

Other approaches for influencing a neural oscillation pattern in a brain are known. US 2018/0236232 discloses methods and a system for acoustic stimulation of brain waves. WO 2019/211845 discloses a device and method for brain stimulation.

US 2019/255350 A1 relates to systems and methods for treating cognitive dysfunction in a subject in need thereof. The system can include eyeglasses, a photodiode positioned to detect ambient light, light sources, and an input device. The system can include a neural stimulation system that retrieves a profile and selects a light pattern having a fixed parameter and a variable parameter. The neural stimulation system can set a value of the variable parameter of the light pattern, construct an output signal, and then provide the output signal to the light sources to direct light towards the fovea.

US 4 883 067 A relates to a method and apparatus for applying a musical feed back signal to the human brain to induce controllable psychological and physiological responses. A signal representing the ongoing electroencephalographic (EEG) signal of a brain preferably is obtained from the electrode location on the scalp known as CZ or P3 in clinical notation. A signal processor converts the ongoing EEG into electrical signals which are converted into music by synthesizers. The music is acoustically fed back to the brain after a time delay calculated to shift the phase of the feedback in order to reinforce specific or desired ongoing EEG activity from the scalp position of interest. The music is comprised of at least one voice that follows the moment-by-moment contour of the EEG in real time to reinforce the desired EEG activity. The music drives the brain into resonance with the music to provide a closed loop or physiological feedback effect.

WO 2021/016624A1 relates to a computer implemented method for slow-wave sleep modulation therapy is provided. The method includes determining a need for sleep wave modulation; and providing the modulation via slow- wave stimulus to a user, wherein the slow-wave stimulus comprises oscillations of a frequency between 0.25 and 8 Hz, and wherein, in response to the slow- wave stimulus, a brain frequency of the user receiving the slow- wave stimulus is modulated closer to the frequency of the slow- wave stimulus than the brain frequency prior to receiving the slow- wave stimulus.

There are disadvantages associated with the prior art approaches taken to date. Some such approaches are limited in terms of the results that can be achieved and/or are costly to implement. Some known approaches require brain implants. Some known approaches have unwanted side effects. Some known approaches require attention and active participation of the user. Moreover, some conventional approaches are not suitable for application in non-clinical settings such as a user's home.

It is an object of the present invention to at least partly mitigate one or more of the above-mentioned problems.

This object is achieved by the methods and systems as defined by the claims.

Accordingly, there is provided a method according to claim 1 of closed-loop modulation of audio data, the method comprising the steps of:
receiving first initial audio data,
receiving a first set of neural oscillation data for a first time period,
obtaining one or more historical neural oscillation data for one or more historical time periods previous to the first time period,
determining first modulated audio data based on the first initial audio data, the first set of neural oscillation data, the one or more historical neural oscillation data, and a configured input-output relationship, and
delivering the first modulated audio data to a user device.

The method may comprise the steps of:
receiving second initial audio data,
receiving a second set of neural oscillation data for a second time period subsequent to the first time period,
obtaining one or more historical neural oscillation data for one or more historical time periods previous to the second time period,
determining second modulated audio data based on the second initial audio data, the second set of neural oscillation data, the one or more historical neural oscillation data, and the configured input-output relationship, and
delivering the second modulated audio data to the user device.

The steps may be repeated iteratively for one or more subsequent sets of audio data.

The audio data may comprise music data. The music data may comprise at least one of MIDI instruction data, MIDI effects data, human voice recording data, and instrumental recording data. The music data may comprise two or more tones, and/or voices, and/or acoustic structures, and the music data may vary over time in a pre-determined manner. The music data may comprise pre-generated music data. The method may comprise the step of generating the music data before receiving the first set of neural oscillation data for the first time period. The audio data may comprise a continuous audio signal.

In this patent specification the term 'music' refers to an ongoing or continuous auditory signal. Music may comprise multiple (at least two) tones and/or voices and/or other acoustic structures. Music may change over time in a predetermined way. For example the pitch and/or rhythm and/or tonality may change over time. In the method of the invention one or more of these changes may be pre-arranged to change dependent on the one or more recorded brain activities. The changes may be pre-arranged to change dependent on the phase(s) of the one or more recorded brainwaves. With the method of the invention the auditory signal may be different each time it is listened to by the user.

For example the audio data may be a piece of piano music where the timing of some or all of the notes are changed dependent on the one or more phases recorded the from one or more brainwaves. For example a bass or baseline of a piece of music may change its volume dependent on the phase recorded from a brainwave.

The first set of neural oscillation data for the first time period may be received from one or more sensors. The first set of neural oscillation data for the first time period may be received from one or more Electroencephalography sensors mountable to a scalp of the user. The method may comprise the step of locating the one or more sensors at one or more desired areas of the head of the user to target neural oscillation activity at one or more corresponding desired areas of the brain of the user. The first set of neural oscillation data may comprise cortical activity. The neural oscillation data may comprise awake neural oscillation data for a user in an awake state.

The first set of neural oscillation data may be phase shifted based on the one or more historical neural oscillation data and the configured input-output relationship to determine the first modulated audio data. The first set of neural oscillation data, and the one or more historical neural oscillation data may be filtered to determine the first modulated audio data. The first set of neural oscillation data, and the one or more historical neural oscillation data may be bandpass filtered to determine the first modulated audio data. The first set of neural oscillation data, and the one or more historical neural oscillation data may be filtered using a finite impulse response filter to determine the first modulated audio data. The finite impulse response filter may have an impulse response of the form: $h \left(t\right) = A cos \left(ωt+φ\right) {e}^{- k ω t} - C$ where
*h(t)* is the impulse response of the filter,
*A* is a real number that sets the closed loop feedback gain,
C is a real number chosen such that the DC gain is zero,
ω is the angular frequency of the neural oscillation to be affected,
t is time,
*k* is a positive real number that determines band width of the filter and a phase shift of each filter,
φ is the phase-shift of the input-output relationship.

For incoming neural oscillation data x(n) sampled at a sampling interval T, the filter may be implemented in a discrete time domain by a convolution given by: $y \left[n\right] = {\sum }_{j = 0}^{N} {b}_{j} . x \left[n-j\right]$ ${b}_{j} = A cos \left(ωjτ+φ\right) {e}^{- kωt} - C$ where C is chosen to ensure that the DC gain is zero: ${\sum }_{j = 0}^{N} {b}_{j} = 0$
*N* is the order of the filter, which spans at least one cycle of the oscillation (i.e. $N > \frac{2 π}{ω τ}$) y[n] is the output of the filter,
bⱼ is the discretised impulse response,
x is the input signal to the filter,
τ is the time interval between successive sampling points.

The first modulated audio data may be determined based on an amplitude parameter of the first set of neural oscillation data, and an amplitude parameter of the one or more historical neural oscillation data.

The first modulated audio data may be determined by applying one or more audio effects to the first initial audio data. The method may comprise the step of altering one or more parameters of the audio effect based on the configured input-output relationship. The first modulated audio data may be determined by altering one or more parameters of audio synthesis based on the configured input-output relationship.

The method may comprise the steps of:
receiving two or more sets of neural oscillation data for the first time period, and
the two or more sets of neural oscillation data, and the one or more historical neural oscillation data are filtered using two or more filters to determine the first modulated audio data with a plurality of audio effects,
each filter being configured to target a different frequency range.

The method may comprise the step of the user device generating audio waves based on the first modulated audio data. The user device may comprise headphones. The method may comprise the step of delivering the audio waves to the user. The audio waves may be delivered to the user to adjust neural oscillation activity of the user. The audio waves may be delivered to the user to enhance or suppress neural oscillation activity of the user.

The method may comprise the step of receiving user selection data. The user selection data may comprise one or more selected frequency ranges. The audio waves may be delivered to the user to adjust neural oscillation activity of the user at the one or more selected frequency ranges. The first modulated audio data may be determined based on the first initial audio data at the one or more selected frequency ranges, the first set of neural oscillation data at the one or more selected frequency ranges, the one or more historical neural oscillation data at the one or more selected frequency ranges, and the configured input-output relationship. The user selection data may comprise one or more selected regions of a brain of a user. The audio waves may be delivered to the user to adjust neural oscillation activity of the user at the one or more selected regions of the brain of the user. The user selection data may comprise a selection of the first initial audio data.

The method comprises the step of determining the configured input-output relationship.

The configured input-output relationship is determined by open-loop calibration of a neural oscillation modulator. The method comprises the steps of:
receiving calibration audio data,
delivering the calibration audio data to a user device,
responsive to the calibration audio data delivered to the user device, receiving calibration neural oscillation data for the user,
determining a phase shift between the calibration audio data and the calibration neural oscillation data, and
the configured input-output relationship is determined based on the phase shift.

The method may comprise the steps of:
obtaining an initial pre-defined input-output relationship,
determining calibration modulated audio data based on the calibration audio data, and the initial pre-defined input-output relationship, and
delivering the calibration modulated audio data to the user device.

The method may comprise the step of configuring a filter of the neural oscillation modulator based on the configured input-output relationship. The method may comprise the step of configuring a finite impulse response filter of the neural oscillation modulator based on the configured input-output relationship. The phase shift between the calibration audio data and the calibration neural oscillation data may be determined at the one or more selected frequency ranges.

The method may comprise determining the modulated audio data, via a synthesiser element, via the steps of:
determining at least one channel, including a respective channel source for each channel whereby at least one channel is a modulatable channel having a modulatable effect that is modulatable responsive to a respective modulation parameter; and
selectively modulating the modulatable effect by selectively varying the respective modulation parameter.

The modulation parameter may be a parameter that varies a music audio signal over time. The music audio signal may be varied continuously over time based on the current phase. The phase may be shifted by the calculated phase shift.

The method may comprise providing a respective pre-recorded or pre-synthesised stream of audio data via each channel source or providing a respective stream of MIDI instructions that specify at least one predetermined characteristic for a sequence of notes via each channel source.

The method may comprise for each modulatable channel, selectively varying a respective modulation parameter in real time responsive to a sensed neural oscillation pattern sensed by a sensor element proximate to a scalp region of the user.

The step of varying a respective modulation parameter may comprise selectively varying a parameter of an audio effect or a MIDI effect or a MIDI instrument or a mixer.

The method may comprise:
providing at least one phase change value for a phase change applicable to at least one respective modulation parameter; and
in real time applying the phase change to the modulation parameter thereby modulating a modulatable effect and generating the modulated audio data responsive thereto.

The method may be non-invasive.

The method may comprise producing a desired influence by boosting or supressing neural oscillation in a desired frequency range by selectively increasing or decreasing oscillatory activity in at least one selected frequency range in at least one area of the brain.

The method of the invention may continually synthesise a music audio signal based on a continuous brain signal. The method may for example use a buffer size of about 5 milliseconds. The method may for example use EEG data sampled every 3 milliseconds. The modulation parameter may change continuously over time. The underlying function used to control and change the modulation parameter may be updated periodically.

It is disclosed but unclaimed that the method may modify a neural oscillation pattern to improve memory or improve relaxation or improve attention or improve sleep in the target user.

It is disclosed but unclaimed that the method may comprise improving symptoms of at least one neurological condition that optionally is ADHD, depression, anxiety or insomnia.

It is disclosed but unclaimed that the method may comprise providing providing a desired therapeutic effect or a desired non therapeutic effect on the target user.

The method may comprise enhancing a desired aesthetic effect of the sound.

The method may comprise modifying the neural oscillation pattern in response to a user selected or clinician selected or composer selected modification option selected by the user or clinician or composer respectively.

Disclosed is a method of improving performance of a physical attribute of a human user comprising any of the steps as described previously.

Disclosed is a method of reducing a perception of anxiety and/or depression and/or stress and/or fatigue comprising any of the steps as described previously.

Also provided but unclaimed is a method of open-loop calibration of a neural oscillation modulator, the method comprising the steps of:
receiving initial audio data,
delivering the initial audio data to a user device,
responsive to the initial audio data delivered to the user device, receiving neural oscillation data for the user,
determining a phase shift between the initial audio data and the neural oscillation data, and determining an input-output relationship for the neural oscillation modulator based on the phase shift.

That method may comprise the steps of:
obtaining an initial pre-defined input-output relationship,
determining modulated audio data based on the initial audio data, and the initial pre-defined input-output relationship, and
delivering the modulated audio data to the user device.

That method may comprise the step of performing cross-spectral analysis of the initial audio data and the neural oscillation data to determine the phase shift between the initial audio data and the neural oscillation data.

That method may comprise the steps of:
performing a Fourier transform of the initial audio data,
performing a Fourier transform of the neural oscillation data, and
the phase shift is determined based on the Fourier coefficient of the initial audio data and complex conjugate of the Fourier coefficient of the neural oscillation data.

That method may comprise the step of configuring a filter of the neural oscillation modulator based on the input-output relationship. The method may comprise the step of configuring a finite impulse response filter of the neural oscillation modulator based on the input-output relationship.

The input-output relationship may comprise a transfer function.

That method may comprise the step of the user device generating audio waves based on the initial audio data. The user device may comprise headphones. The method may comprise the step of delivering the audio waves to the user.

That method may comprise the step of receiving user selection data. The user selection data may comprise one or more selected frequency ranges. The phase shift between the initial audio data and the neural oscillation data may be determined at the one or more selected frequency ranges. The neural oscillation data may comprise awake neural oscillation data for a user in an awake state.

That method may comprise the steps of determining at least a respective phase change and/or a respective amplitude change associated with a whole or a portion of a sound creation pathway between the initial audio data provided to the user device and a sensed neural oscillation pattern sensed via at least one sensor element proximate to a scalp region of the user.

That method may comprise performing cross spectral analysis between a calibration pattern associated with the initial audio data and the neural oscillation data pattern.

That method may comprise determining a transfer function associated with the sound creation pathway for each of at least one selected frequency range in the initial audio data, a phase shift of the transfer function in each frequency range comprising a respective phase change associated with the sound creation pathway.

That method may comprise for each frequency of interest, each frequency of interest being associated with a respective selected frequency range, providing a respective filter element having a pass band around the frequency of interest and a phase shift equal and opposite to a phase associated with the determined transfer function for that frequency range.

That method may comprise:
providing a set of filter elements for all frequency ranges, each filter element in the set being respectively associated with a respective one of all frequencies of interest; and
determining the transfer function responsive to said set.

That method may comprise determining the transfer function by selecting a sign of a filter gain for each filter element in the set of filter elements responsive to a selection of if the neural oscillation in at least one frequency range associated with the set is to be boosted or supressed.

That method may comprise:
providing each filter element as a finite impulse response filter with an impulse response of the form: $h \left(t\right) = A cos \left(ωt+φ\right) {e}^{- k ω t} - C$
where
*h*(*t*) is the impulse response of the filter,
*A* is a real number that sets the closed loop feedback gain,
*C* is a real number chosen such that the DC gain is zero,
ω is the angular frequency of the neural oscillation to be affected,
t is time,
*k* is a positive real number that determines band width of the filter and a phase shift of each filter,
φ is the phase-shift of the input-output relationship.

That method may comprise:
for incoming neural oscillation data x(n) sampled at a sampling interval T, implementing a filter in a discrete time domain by a convolution given by: $y \left[n\right] = {\sum }_{j = 0}^{N} {b}_{j} . x \left[n-j\right]$ ${b}_{j} = A cos \left(ωjτ+φ\right) {e}^{- kωt} - C$ where C is chosen to ensure that the DC gain is zero: ${\sum }_{j = 0}^{N} {b}_{j} = 0$
*N* is the order of the filter, which spans at least one cycle of the oscillation (i.e. $N > \frac{2 π}{ω τ}$) y[n] is the output of the filter,
bⱼ is the discretised impulse response,
x is the input signal to the filter,
*τ* is the time interval between successive sampling points.

That method may comprise sensing a neural oscillation data pattern via a Magnetoencephalography (MEG) sensor array or an Electroencephalography (EEG) sensor array.

That audio data may be audio sound in a frequency range of around 20Hz to 20kHz and/or infrasound in the frequency range below around 20Hz.

That method may comprise the step of locating one or more sensors at one or more desired areas of the head of the user to target neural oscillation activity at one or more corresponding desired areas of the brain of the user.

In one embodiment of the method of the invention, a predetermined set of audio signals may be continuously modulated in real-time based on the listener's brain-activity recorded in real-time. The method may be a continuous closed-loop process working in real-time. The method may operate on a millisecond basis at a speed similar to the speed of human hearing.

In one embodiment of the method of the invention, there is no need to store user data such as the recorded brain-activity data over a long period of time. The method of the invention may operate in real-time based on a small number of most recent samples of recorded brain-activity data.

In one embodiment of the method of the invention, the method does not require a specific action or concentration on the part of the user. The predetermined audio signal may be for example the user's favourite piece of music. The method ensures that this piece of music will sound somewhat different to the user because of the real-time modulation of at least one aspect of the piece of music, such as the volume of the bass-line, and/or the timing of certain tones, and the like. To minimize computational power and to achieve a maximally positively sounding listening experience, the details of the modulation may be predetermined. It may therefore be sufficient to coordinate the timing of the execution of the modulation in real-time based on the recorded brain signals.

The audio signal may be pre-generated music.

The invention also provides in a further aspect a system for closed-loop modulation of audio data, the system comprising:
a neural oscillation modulator for receiving first initial audio data, receiving a first set of neural oscillation data for a first time period, and obtaining one or more historical neural oscillation data for one or more historical time periods previous to the first time period,
the neural oscillation modulator being configured to determine first modulated audio data based on the first initial audio data, the first set of neural oscillation data, the one or more historical neural oscillation data, and a configured input-output relationship,
the neural oscillation modulator being configured to deliver the first modulated audio data to a user device.

The system comprises means to perform the method of the invention.

The system may comprise a portable consumer device.

In a further aspect of the invention there is provided a system for open-loop calibration, the system comprising:
an audio processor for receiving initial audio data, and delivering the initial audio data to a user device, and
a calibrator for, responsive to the initial audio data delivered to the user device, receiving neural oscillation data for a user,
the calibrator configured to determine a phase shift between the initial audio data and the neural oscillation data, and
the calibrator configured to determine an input-output relationship for the neural oscillation modulator based on the phase shift.

The system comprises means to perform the method of the invention.

The invention also provides in another aspect a computer program product comprising computer program code capable of causing a computer system to perform a method of the invention when the computer program product is run on a computer system.

Certain embodiments of the present invention utilise closed-loop feedback to continuously measure effective brain oscillation activity in a brain of a target user and use the continuously measured signals from one or more regions of a brain to continuously modulate one or more elements of music played to the user. Such music stimulates by either enhancing or suppressing the generation of desired patterns in the neural oscillation patterns.

By determining the modulated audio data based on the first set of neural oscillation data, and also based on the historical neural oscillation data, this enables the method of the invention to achieve enhanced accuracy and control of the user's neural oscillation activity by means of the modulated audio data. In particular the multiple preceding brain signal time intervals allow precise control to be achieved to account for any differences in hardware being used, such as the headset being worn by the user, or the sound generation device, or the communication network capabilities over which the data is transmitted.

In one embodiment the invention addresses the technical problem of how to enable a catalogue of brain-responsive music to be composed, calibrated, and delivered to a user, for example delivered through an on-line streaming platform and mobile device. The invention provides a system for a music composer to generate a catalogue of brain-responsive music and distribute the music to a user via streaming as digital files that may be processed on a variety of mobile devices.

Certain embodiments of the present invention provide a method of selectively modifying a neural oscillation pattern in a brain of a target user. An overall neural oscillation pattern or a regional neural oscillation pattern or multiple regional neural oscillation patterns in a brain of a user can be varied relative to an initial state.

Certain embodiments of the present invention provide music as a stimulation modality. Music is a complex acoustic stimulus that can activate wide-spread areas of brain. Different elements within music (pitch, volume, tone, timbre, stereo width or the like) can be used to entrain brain oscillations at different frequencies. The invention may enable any of the elements to be selected for modulation in a frequency which matches a specific brain oscillation. The elements may be selected based on acoustic factors and/or the elements may be selected based on efficacy factors. The invention may enable a single voice to be modulated for such an effect.

Certain embodiments of the present invention provide a system and method to deliver 'closed-loop' music. Feedback loops, either positive or negative, may be provided between brain signals at specific frequencies and the continuous modulation of acoustic elements within the music. The result is that brain signals at different frequencies in different parts of the brain may be selectively enhanced or suppressed.

Certain embodiments of the present invention may be used either to have a desired mental and/or therapeutic effect on the listener, or to enhance an aesthetic effect that is desired by the composer. In the first use case, the listener or other decision maker decides which oscillations should be boosted or suppressed to achieve the desired mental and/or therapeutic effect.

For example the following are some possible relations between brain oscillations and mental and/or therapeutic effects:

| Frequency name | Example frequency range (Hz) | Related effect |
|---|---|---|
| Gamma | >35 Hz | Concentration |
| Beta | 12-35 Hz | Attention to external events, anxiety |
| Alpha | 8-12 Hz | Relaxation, passive attention |
| Theta | 4-8 Hz | Relaxation, breathing |
| Delta | 0.5 - 4 Hz | Sleep |

The present invention may be used to increase or decrease these frequencies to boost or supress the related effects. The frequency ranges listed above are merely examples. It will be appreciated that the exact frequency ranges may vary across subjects, and/or over time, and/or across different regions of the brain.

In the second use case, the composer decides which oscillations should be boosted and/or suppressed (potentially dynamically as the piece develops) to enhance the aesthetic effect of the music.

Certain embodiments of the present invention confer flexibility to a composer to be able to use whatever elements of music are appropriate to their creative vision. The invention provides a system that allows a multitude of different elements in music to be modulated and provides a format for storing and distributing the instructions for generating such music. The file format may include the information required to generate the modulated parts of the music in combination with the user's brain signals. This file format may be audio files, MIDI note instructions, instructions about audio effects that are applied to these components, how these audio effects are to be modulated by brain signals, and how the individual elements are to be mixed into the non-modulated parts of the music to produce a single stereo audio output.

Certain embodiments of the present invention provide for calibration of a system to a specific user such as a human listener, using specific hardware that may have variable operating characteristics due to manufacturing tolerances. In particular certain embodiments of the present invention accommodate latency and phase change introduced by the hardware implementation to maximise the likelihood that a desired effect, such as suppression or boosting, of a desired oscillatory activity in one or more different frequency bands, for example alpha, beta or feta bands, in different brain areas is achieved.

The method of the invention may use music as the stimulation modality. Music is a complex acoustic stimulus that may activate wide-spread areas of brain. Different elements within music, such as pitch, volume, tone, timbre, stereo width, may entrain brain oscillations at different frequencies. The perception of music may be influenced by its timing relative to brain oscillations, the modulation of brain oscillations may be related to enjoyment of the music, and music may have a mental and/or therapeutic effect in many conditions. The system and method of the invention deliver 'closed-loop' music with feedback loops, either positive or negative, between brain signals at specific frequencies and the continuous modulation of acoustic elements within the music. This results in brain signals at different frequencies in different parts of the brain being selectively enhanced or suppressed. In this manner the invention may be used to have a desired therapeutic effect on the listener, or to enhance the aesthetic effect that is desired by the composer. The listener may decide which oscillations should be boosted or suppressed to achieve the desired therapeutic effect. The composer may decide which oscillations should be boosted and/or suppressed, potentially dynamically as the piece develops, to enhance the aesthetic effect of the music.

The method of the invention determines the modulated audio data based on the initial audio data, the first set of neural oscillation data, the one or more historical neural oscillation data, and the configured input-output relationship. The input signals are the initial audio data, the first set of neural oscillation data, and the one or more historical neural oscillation data. The output signal is the modulated audio data. The configured input-output relationship is predetermined by an initial calibration technique so that the resulting effect is specially personalised for the target user. Because the configured input-output relationship is predetermined to be personalised for the target user, this results in enhanced control of the neural oscillation patterns in the brain of the target user. In particular it has been found that by personalising the configured input-output relationship for each target user, this allows more precise sound to be generated and delivered to the target user. Accordingly the neural oscillation pattern in the brain of the target user may be accurately controlled to achieve the desired therapeutic or non-therapeutic effect.

By performing the calibration, the invention may account for any differences in hardware platforms that a user may be using, for example different transmission latencies for wireless headphones. The invention may account for any differences in any audio elements that a composer may wish to modulate, for example volume, pitch, or the like which may influence the brain signals in different ways. The invention may account for any differences in brain regions sensed by a particular recording electrode configuration, for example the latency of the brain response may depend on which brain region is being recorded from. The invention may account for any individual differences in a response of a specific user's brain, for example even if hardware being used is the same, and the modulated elements are the same, and the electrodes are placed in the same location, the brain signals may vary across individuals due to differences in neuroanatomy.

To take into account any variations in the hardware or software aspects of the sound generator being used to generate the sound, the method of the invention determines the configured input-output relationship specific to the sound generator element also. In this manner the neural oscillation pattern in the brain of the target user may be controlled as desired regardless of any variations in the equipment being used to generate the sound. Similarly to take into account any variations in the hardware or software aspects of the communications network being used to transmit the modulated audio data, the method of the invention determines the configured input-output relationship based on a predetermined profile specific to the communications network also. In this manner the neural oscillation pattern in the brain of the target user may be controlled as desired regardless of any variations in the equipment being used to transmit the signals. All of these variations collectively lead to a phase change between the audio data signal and the brain response at the frequency of interest to be controlled. By calibrating this phase change and applying the calibrated phase change within the closed-loop system, these variations may be accounted for with a single phase shift. In further detail the configured input-output relationship alters the closed-loop phase-shift at the target frequency to be 0 degrees for positive feedback to boost the brain signal, or 180 degrees for negative feedback to suppress the brain signal.

The system of the invention is initially calibrated to a specific user and to the specific hardware being operated by a user. This calibration is performed in advance for any of the elements the composer or the user has decided to exploit. This calibration will take account of the phase and polarity relationship between the applicable musical elements and the evoked brain oscillations. This calibration will also take into account the latencies in the hardware, since this may be the user's own device, for example a mobile telephone, which may have different specifications. This calibration will also enable the user or composer to preselect frequencies of interest and whether to boost or suppress these oscillations. For the music to have the desired effect on the brain oscillations, the closed-loop feedback system is calibrated. This calibration adjusts for differences in the hardware configuration of users, for example different latencies of recording/processing and audio generation, differences in the location of recording electrodes, differences in brain responses to various elements of music, differences in brain responses between different individuals. This calibration also defines the frequency of brain oscillations of interest, for example by identifying the frequencies of normal or pathological activity in the brain, or these may be prespecified by the composer or listener.

The invention employs open-loop calibration in which a known audio signal is delivered to a user and the brain response is measured. Based on the brain response the phase shift is calculated, and the filter is configured using the calculated phase shift. The invention employs the FIR filter which receives multiple previous time period signals, and mixes the signals to calculate the optimum future signal. In further detail the invention provides the means to determine how the multiple preceding time period brain signals should be mixed to determine the optimum future time period modulation signal.

The method of the invention may be applied to an existing piece of music by a user. The method of the invention may also be applied to a piece of music newly created for this invention.

According to another aspect of the present invention there is provided a system for closed-loop modulation of audio data, the system comprising:
a neural oscillation modulator for receiving initial audio data, receiving a first set of neural oscillation data for a first time period, and obtaining one or more historical neural oscillation data for one or more historical time periods previous to the first time period,
the neural oscillation modulator being configured to determine modulated audio data based on the initial audio data, the first set of neural oscillation data, the one or more historical neural oscillation data, and a configured input-output relationship.

Aptly, the neural oscillation modulator is further configured to deliver the modulated audio data to a user.

According to another aspect of the present invention there is provided a system for closed-loop modulation of audio data, the system comprising:
an electrical oscillation modulator for receiving initial audio data, receiving a first set of electrical oscillation data for a first time period, and obtaining one or more historical electrical oscillation data for one or more historical time periods previous to the first time period,
the electrical oscillation modulator being configured to determine modulated audio data based on the initial audio data, the first set of electrical oscillation data, the one or more historical electrical oscillation data, and a configured input-output relationship, and deliver the modulated audio data to a user device.

Aptly the electrical oscillation modulator is a neural oscillation modulator. Aptly the electrical oscillation data is neural oscillation data. Aptly the user is a human user.

According to another aspect of the present invention there is provided a non-invasive method of closed-loop modulation of audio data, the method comprising the steps of:
receiving initial audio data,
receiving a first set of neural oscillation data for a first time period,
obtaining one or more historical neural oscillation data for one or more historical time periods previous to the first time period,
determining modulated audio data based on the initial audio data, the first set of neural oscillation data, the one or more historical neural oscillation data, and a configured input-output relationship, and
delivering the modulated audio data to a user device.

According to another aspect of the present invention there is provided a non-invasive method of open-loop calibration of a neural oscillation modulator, the method comprising the steps of:
receiving initial audio data,
delivering the initial audio data to a user device,
responsive to the initial audio data delivered to the user device, receiving neural oscillation data for a user,
determining a phase shift between the initial audio data and the neural oscillation data, and
determining an input-output relationship for the neural oscillation modulator based on the phase shift.

Certain embodiments of the present invention provide a non-invasive method. Certain embodiments of the present invention provide a non-surgical method. Aptly the method of closed-loop modulation of audio data does not comprise a surgical step. Aptly the method of open-loop calibration of a neural oscillation modulator does not comprise a surgical step.

According to another aspect of the present invention there is provided a non-invasive system for closed-loop modulation of audio data, the system comprising:
a neural oscillation modulator for receiving initial audio data, receiving a first set of neural oscillation data for a first time period, and obtaining one or more historical neural oscillation data for one or more historical time periods previous to the first time period,
the neural oscillation modulator being configured to determine modulated audio data based on the initial audio data, the first set of neural oscillation data, the one or more historical neural oscillation data, and a configured input-output relationship, and deliver the modulated audio data to a user device.

Certain embodiments of the present invention provide a non-invasive system. Certain embodiments of the present invention provide a non-surgical system. Aptly the system for closed-loop modulation of audio data does not comprise a surgical step.

Certain embodiments of the present invention provide non-invasive methods for neuromodulation of a user. The neuromodulation may result in a desired therapeutic effect in the user. The neuromodulation may result in a desired non-therapeutic effect in the user.

Certain embodiments of the present invention provide a system for non-invasive neuromodulation of a user. The neuromodulation may result in a desired therapeutic effect in the user. The neuromodulation may result in a desired non-therapeutic effect in the user.

Certain embodiments of the present invention provide a method further comprising a desired therapeutic effect. Certain embodiments of the present invention may result in selectively modifying a neural oscillation pattern in a user resulting in a desired therapeutic effect. Certain embodiments of the present invention provide a method for the treatment and/or prevention of a neurological disease or condition, or alleviating symptoms associated with the disease or condition.

Certain embodiments of the present invention provide a system for selectively modifying a neural oscillation pattern in a user resulting in a desired therapeutic effect. Certain embodiments of the present invention provide a system for the treatment and/or prevention of a neurological disease or condition, or alleviating symptoms associated with the disease or condition.

Non-limiting examples of neurological diseases and conditions include attention deficit hyperactivity disorder (ADHD), depression, anxiety, dementia, schizophrenia, ataxia, headaches, migraines, Bell's Palsy, Parkinson disease, bi-polar disorder, insomnia, Alzheimer disease, encephalitis, mood disorders, epilepsy, seizures, mental health disorders, stroke, traumatic brain injury, hypoxic brain injury, muscular dystrophy and neuromuscular diseases.

In certain embodiments the neurological disease is attention deficit hyperactivity disorder. In certain embodiments the neurological disease is depression. **In** certain embodiments the neurological disease is anxiety. In certain embodiments the neurological disease is insomnia.

In certain embodiments the user is in a pathological state. In certain embodiments the user is displaying clinical signs of disease, infection or illness. In certain embodiments the user has a neurological disease or condition.

Certain embodiments of the present invention provide a method of reducing a perception of anxiety in a user. Certain embodiments of the present invention provide a system for reducing a perception of anxiety in a user. Aptly the user does not have an anxiety disorder, but rather has been subjected to a transitory circumstance causing a perception of anxiety, for example an uncertain situation.

Certain embodiments of the present invention provide a method of reducing a perception of depression in a user. Certain embodiments of the present invention provide a system for reducing a perception of depression in a user. Aptly the user does not have pathological depression, but rather has been subjected to a transitory circumstance causing a perception of depression, for example a situation that makes them feel sad.

Certain embodiments of the present invention provide a method of reducing a perception of stress in a user. Certain embodiments of the present invention provide a system for reducing a perception of stress in a user. Aptly the user does not have pathological stress, but rather has been subjected to a transitory circumstance causing a perception of stress, for example a stressful situation.

Certain embodiments of the present invention provide a method of reducing a perception of fatigue in a user. Certain embodiments of the present invention provide a system for reducing a perception of fatigue in a user. Aptly the user does not have pathological fatigue, but rather has been subjected to a transitory circumstance causing a perception of fatigue, for example the performance of exercise.

Certain embodiments of the present invention provide a method of reducing a perception of anxiety and/or depression and/or stress and/or fatigue in a user. Certain embodiments of the present invention provide a system for reducing a perception of anxiety and/or depression and/or stress and/or fatigue in a user. Aptly the user does not have a mental condition, but rather is in a transitory physiological condition caused by transient natural circumstance.

According to another aspect of the present invention there is provided a method of closed-loop modulation of audio data, the method comprising the steps of:
receiving initial audio data,
receiving a first set of neural oscillation data for a first time period,
obtaining one or more historical neural oscillation data for one or more historical time periods previous to the first time period,
determining modulated audio data based on the initial audio data, the first set of neural oscillation data, the one or more historical neural oscillation data, and a configured input-output relationship, and
delivering the modulated audio data to a device for a healthy user.

According to another aspect of the present invention there is provided a method of open-loop calibration of a neural oscillation modulator, the method comprising the steps of:
receiving initial audio data,
delivering the initial audio data to a device for a healthy user,
responsive to the initial audio data delivered to the device for the healthy user, receiving neural oscillation data for the healthy user,
determining a phase shift between the initial audio data and the neural oscillation data, and
determining an input-output relationship for the neural oscillation modulator based on the phase shift.

According to an aspect of the present invention there is provided a system for closed-loop modulation of audio data, the system comprising:
a neural oscillation modulator for receiving initial audio data, receiving a first set of neural oscillation data for a first time period, and obtaining one or more historical neural oscillation data for one or more historical time periods previous to the first time period,
the neural oscillation modulator being configured to determine modulated audio data based on the initial audio data, the first set of neural oscillation data, the one or more historical neural oscillation data, and a configured input-output relationship, and deliver the modulated audio data to a device for a healthy user.

In certain embodiments the user is healthy. In certain embodiments the user is a person in a good physical or mental condition. In certain embodiments the user is not displaying clinical signs of disease, infection, or illness. In certain embodiments the user is not in a pathological state. In certain embodiments the user is not displaying clinical signs of a neurological disease or condition. In certain embodiments the user does not have a neurological disease or condition.

According to another aspect of the present invention there is provided a method of closed-loop modulation of audio data, the method comprising the steps of:
receiving initial audio data,
receiving a first set of neural oscillation data for a first time period,
obtaining one or more historical neural oscillation data for one or more historical time periods previous to the first time period,
determining modulated audio data based on the initial audio data, the first set of neural oscillation data, the one or more historical neural oscillation data, and a configured input-output relationship, and
delivering the modulated audio data to a user device,
wherein the modulated audio data results in a desired non-therapeutic effect in the user.

Certain embodiments of the present invention provide a method which results in a desired non-therapeutic effect in a user. Certain embodiments of the present invention provide a method of selectively modifying a neural oscillation pattern in a user resulting in a desired non-therapeutic effect. Certain embodiments of the present invention provide a method for the non-therapeutic improvement and/or enhancement in a healthy user.

Certain embodiments of the present invention provide a system for selectively modifying a neural oscillation pattern in a user resulting in a desired non-therapeutic effect. Certain embodiments of the present invention provide a system for non-therapeutic improvement and/or enhancement in a healthy user.

Certain embodiments of the present invention provide a method for improving attentiveness in a user. Certain embodiments of the present invention provide a system for improving attentiveness in a user. Aptly the user does not have an attention disorder, for example attention deficit hyperactivity disorder.

Certain embodiments of the present invention provide a method for improving sleep in a user. Certain embodiments of the present invention provide a system for improving sleep in a user. Aptly the user does not have a sleep disorder, for example insomnia.

Certain embodiments of the present invention provide a method for improving memory in a user. Certain embodiments of the present invention provide a system for improving memory in a user. Aptly the user does not have a memory disorder, for example dementia.

Certain embodiments of the present invention provide a method for improving relaxation in a user. Certain embodiments of the present invention provide a system for improving relaxation in a user. Aptly the user does not have an anxiety disorder, for example generalized anxiety disorder.

Certain embodiments of the present invention provide a method for improving relaxation and/or sleep and/or memory and/or relaxation in a user. Certain embodiments of the present invention provide a system for improving relaxation and/or sleep and/or memory and/or relaxation in a user. Aptly the user is not in a pathological state, but rather is healthy.

Without being bound by theory, the healthy user is not likely to develop a pathological disorder because of the absence of selective modification of their neural oscillation pattern. Aptly certain embodiments of the present invention, which provide a method of closed-loop modulation of an audio signal cannot be considered prophylactic. Aptly certain embodiments of the present invention, which provide a method of selectively modifying a neural oscillation pattern in a healthy user cannot be considered prophylactic. Aptly certain embodiments of the present invention, which provide a system for closed-loop modulation of an audio signal cannot be considered prophylactic. Aptly certain embodiments of the present invention, which provide a system for selectively modifying a neural oscillation pattern in a healthy user cannot be considered prophylactic.

Embodiments of the present invention will now be described hereinafter, by way of example only, with reference to the accompanying drawings in which:
Figure 1 illustrates a human target user wearing a headset support that supports headphones and sensors;
Figure 2 illustrates the workflow for providing sound to a user responsive to the user's neural oscillations;
Figure 3 illustrates a workflow for applying audio effects to a music file;
Figure 4 illustrates a workflow for modifying a music file responsive to neural oscillations;
Figure 5 illustrates a closed-loop EEG configuration;
Figure 6 illustrates a closed-loop MEG configuration;
Figure 7 illustrates a power spectrum for EEG power for different phase shifts;
Figure 8 illustrates the relative power with respect to a control for open-loop and closed-loop feedback;
Figure 9 illustrates how closed-loop musical brain stimulation controlled by EEG using a filter frequency of 5Hz is capable of modulating theta-band oscillations in a brain;
Figure 10 illustrates how closed-loop musical brain stimulation controlled by EEG using a filter frequency of 10Hz is capable of modulating alpha-band oscillations in a brain;
Figure 11 illustrates results from an MEG example in which closed-loop musical stimulation was controlled by brain signals from the right temporal cortex;
Figure 12 illustrates results from an MEG example in which closed-loop musical stimulation was controlled by brain signals from the central prefrontal cortex;
Figure 13 illustrates a correlation of closed-loop optimal phase shifts versus open-loop phase shifts;
Figure 14 is a schematic illustration of a system according to the invention for closed-loop modulation of audio data;
Figure 15 is a block diagram illustrating operation of the system of Figure 14;
Figure 16 is a block diagram illustrating closed-loop operation of the system of Figure 14, and a closed-loop transfer function;
Figure 17 is a block diagram illustrating open-loop calibration of the system of Figure 14, and an open-loop transfer function;
Figures 18 and 19 illustrate average changes in EEG power obtained following use of a system according to the invention for closed-loop modulation of audio data; and
Figures 20 and 21 illustrate heart rate variability obtained following use of a system according to the invention for closed-loop modulation of audio data.

In the drawings like reference numerals refer to like parts.

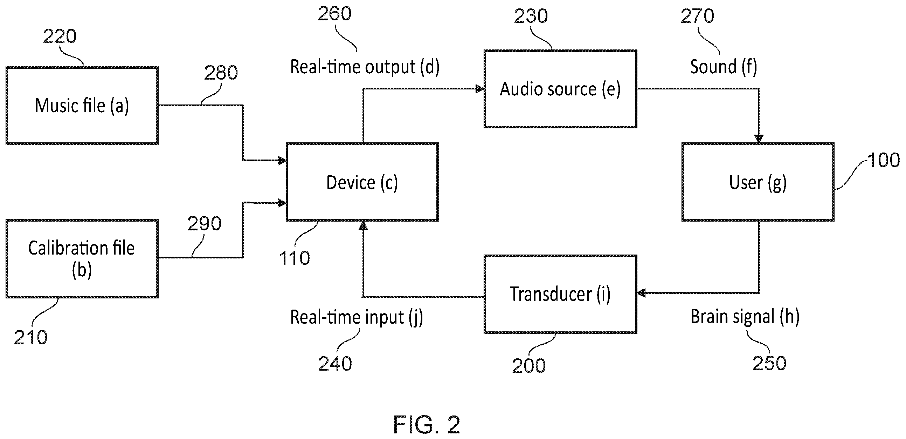

Referring to the drawings and initially to Fig. 2 thereof, there is illustrated a system according to the invention for closed-loop modulation of audio data. The system includes a neural oscillation modulator 110.

The neural oscillation modulator 110 receives initial audio data 220 selected by a user 100. In this case the audio data 220 comprises music data in the form of a continuous audio signal. The music data may include MIDI instruction data, MIDI effects data, or human voice recording data, or instrumental recording data.

The neural oscillation modulator 110 receives one or more frequency ranges selected by the user, and one or more regions of a brain selected by the user.

The neural oscillation modulator 110 receives a first set of neural oscillation data 240 for a first time period. In this case the first set of neural oscillation data 240 is the current or most recent set of neural oscillation data available for the user, and the first time period relates to the current or most recent time period being analysed. The current neural oscillation data 240 includes information relating to brain activity of the user. The current neural oscillation data 240 for the current time period may be received from a plurality of sensors 200. In this case the current neural oscillation data 240 for the current time period is received from a plurality of Electroencephalography sensors 200 mounted to a scalp of the user.

However it will be appreciated that the first set of neural oscillation data may alternatively be an earlier set of neural oscillation data and is not necessarily the current or most recent set available for the user. Similarly the first time period may relate an earlier time period and is not necessarily the current or most recent time period.

The neural oscillation modulator 110 obtains historical neural oscillation data for a plurality of historical time periods previous to the current time period.

The neural oscillation modulator 110 determines modulated audio data 260 based on the initial audio data 220 at the selected frequency ranges, the current neural oscillation data 240 at the selected frequency ranges, the plurality of historical neural oscillation data at the selected frequency ranges, and a pre-configured input-output relationship 210. The current neural oscillation data 240 is phase shifted based on the plurality of historical neural oscillation data and the pre-configured input-output relationship 210 to determine the modulated audio data 260. In this case an amplitude parameter of the current neural oscillation data 240, and an amplitude parameter of the plurality of historical neural oscillation data are bandpass filtered using a finite impulse response filter to determine the modulated audio data 260.

In one possible example embodiment of the invention the finite impulse response filter may have an impulse response of the form: $h \left(t\right) = A cos \left(ωt+φ\right) {e}^{- k ω t} - C$ where
*h(t)* is the impulse response of the filter,
*A* is a real number that sets the closed loop feedback gain,
*C* is a real number chosen such that the DC gain is zero,
ω is the angular frequency of the neural oscillation to be affected,
*t* is time,
*k* is a positive real number that determines band width of the filter and a phase shift of each filter,
φ is the phase-shift of the input-output relationship.

In one possible example embodiment of the invention, for incoming neural oscillation data x(n) sampled at a sampling interval T, the filter may be implemented in a discrete time domain by a convolution given by: $y \left[n\right] = {\sum }_{j = 0}^{N} {b}_{j} . x \left[n-j\right]$ ${b}_{j} = A cos \left(ωjτ+φ\right) {e}^{- kωt} - C$ where C is chosen to ensure that the DC gain is zero: ${\sum }_{j = 0}^{N} {b}_{j} = 0$
*N* is the order of the filter, which spans at least one cycle of the oscillation (i.e. $N > \frac{2 π}{ω τ}$) y[n] is the output of the filter,
bⱼ is the discretised impulse response,
x is the input signal to the filter,
τ is the time interval between successive sampling points.

It will be appreciated that the modulated audio data 260 may be determined using a variety of possible approaches. For example the current neural oscillation data 240, and the plurality of historical neural oscillation data may be convolved with a kernel to determine the modulated audio data 260.

The modulated audio data 260 may be determined by applying one or more audio effects to the initial audio data 220. One or more parameters of the audio effects may be altered based on the output determined by the pre-configured input-output relationship 210. The modulated audio data 260 may be determined by altering one or more parameters of audio synthesis based on the pre-configured input-output relationship 210.

The neural oscillation modulator 110 delivers the modulated audio data 260 to a user device, for example headphones. The user device generates audio waves based on the modulated audio data 260, and delivers the audio waves to the user 100. The audio waves may be delivered to the user to adjust neural oscillation activity of the user at the selected frequency ranges and at the selected regions of the brain of the user, for example to enhance or suppress neural oscillation activity of the user.

In this case the pre-configured input-output relationship 210 is provided in the form of a transfer function. The input-output relationship 210 is pre-determined by performing an open-loop calibration of the neural oscillation modulator 110.

To calibrate the neural oscillation modulator 110, the system receives calibration audio data, and obtains an initial pre-defined input-output relationship. The system determines calibration modulated audio data based on the calibration audio data, and the initial pre-defined input-output relationship, and then delivers the calibration modulated audio data to the user device. The user device generates audio waves based on the calibration modulated audio data, and delivers the audio waves to the user 100. In response to the audio waves being delivered to the user, the system receives calibration neural oscillation data for the user.

The system determines a phase shift between the calibration audio data and the calibration neural oscillation data at the selected frequency ranges. In this case cross-spectral analysis of the calibration audio data and the calibration neural oscillation data is performed to determine the phase shift between the calibration audio data and the calibration neural oscillation data. In particular a Fourier transform of the calibration audio data is performed. A Fourier transform of the calibration neural oscillation data is performed. The phase shift is then determined based on the Fourier coefficient of the calibration audio data and complex conjugate of the Fourier coefficient of the calibration neural oscillation data. The phase of the cross-spectrum indicates what the phase relationship between the two signals is.

The input-output relationship 210 is determined based on the phase shift. In particular the phase-shift of the input-output relationship 210 is configured so that the overall closed-loop phase shift is 0 degrees for boosting of the brain signal or 180 degrees for suppressing the brain signal.

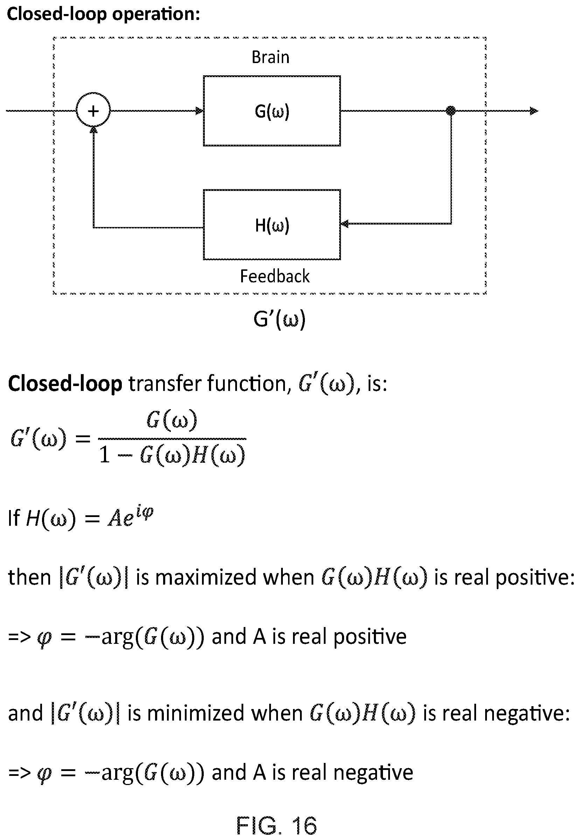

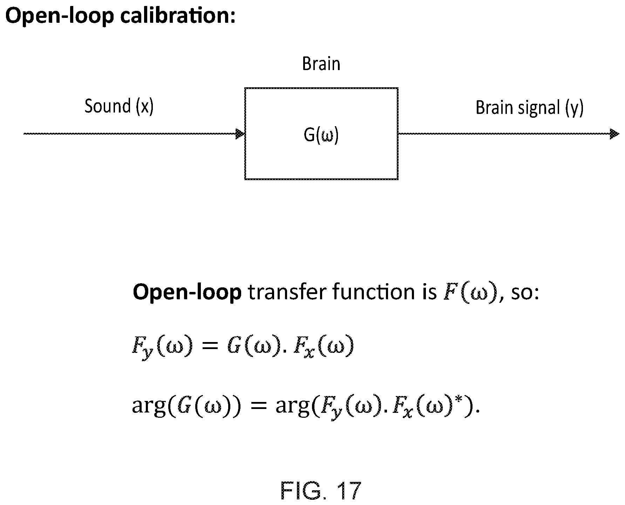

This aspect of determining the phase shift between the calibration audio data and the calibration neural oscillation data is illustrated in further detail in Figs. 16 and 17.

The finite impulse response filter of the neural oscillation modulator 110 may be configured based on the input-output relationship 210.

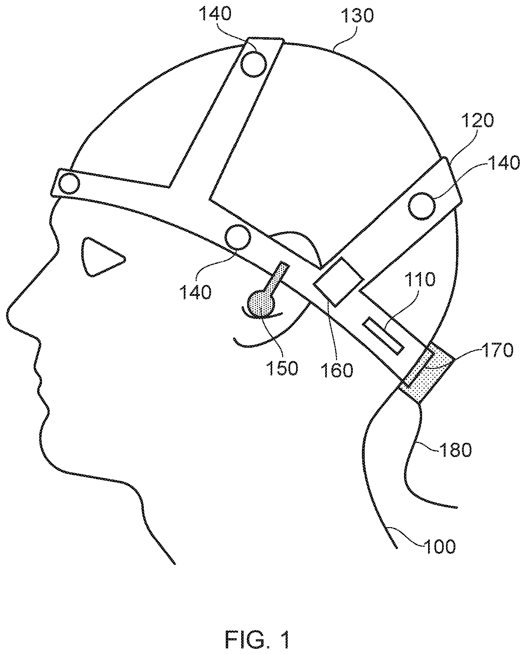

Figure 1 illustrates the user 100 wearing a device 110. In the present embodiment, the device 110 is a headset device that comprises a frame 120 that extends across a scalp 130 of the user 100, that is to say substantially along a coronal plane, and from the forehead of the user 100 to the occiput. However, it will be understood that in alternative embodiments, the frame 120 may extend along the scalp of the user substantially along a sagittal plane. Moreover, it will be understood that instead of a frame 120, the headset device 110 may comprise adjustable straps that contact the headset device 110 to the user in the same or a similar manner. Furthermore, it will be understood that the device 110 may communicate with a smartphone, laptop, desktop computer, or other computing device. Alternatively the device 110 may be provided as part of or embedded within a smartphone, laptop, desktop computer, or other computing device. Sensors 140 are located proximate to the user's head or scalp at various locations on the headset device 110. In the present embodiment, the sensors 140 measure fluctuations in an electric field, or equivalently changes in an electric potential, induced by brain activity. That is to say, the sensors 140 measure neural oscillations, also known as 'brain waves', of the user's brain. For example, the sensors 140 can measure the neural oscillations of a user responsive to stimulus provided to the user, such as auditory stimulus provided by the headphone 150. In alternative embodiments, the sensors 140 measure fluctuations in a magnetic field induced by brain activity to determine the neural oscillations of the user 100. The neural oscillation measurements of the user 100 measured by the sensors 140 are processed by a processing element 160. The processing element 160 may comprise a memory element for storing digital data. The processing element 160 is suitable for executing instructions such as a computer programme or algorithm. For example, the processing element 160 can process neural oscillations responsive to sound played to the user via headphones 150. In accordance with the present invention, sound comprises any effect on the environment proximate to the user 100 that induced auditory stimulation in the user 100. For example, sound may include sound waves within the frequency range of 20 Hz to 20 kHz. For example, sound may comprise music. According to an algorithm, the processing unit 160 may modify a digital musical file corresponding to the music being played such that when music corresponding to the modified musical file is played to the user 100, a desired change in the neural oscillations of the user 100 is induced.

In an alternative embodiment of the invention the sensors 140 may be affixed to the scalp of the user by adhesive means without any structural frame.

The device 110 also comprises a peripheral adapter 170 by which external devices may be connected to the headset. For example, the peripheral adapter 170 may comprise a network adapter that enables the headset to be connected to a computer network or the internet wirelessly or by a cable 180.

The sensors 140 may be provided in the form of electrodes adhesively affixed to the forehead and behind the ear of the user 100.

The sensors 140 may be implanted below the scalp or above the surface of the brain in the form of electrocorticogram-electrodes implanted subdurally or epidurally.

The brain regions that may be controlled will depend on the specific locations of the sensors 140, as will the latencies of the brain responses. The invention accounts for any such latencies by means of the calibration process.

It will be appreciated that the system and computer program of the invention are compatible for use with a variety of different types of headset.

The device 110 may include an amplifier to amplify the brain signals. The device 110 may include a filter to filter the brain signals, for example to remove line noise at 50/60Hz.

The device 110 may transmit the brain signals to a computer system. The computer system may include a mobile device, such as a tablet device or a smartphone device. For safety reasons, the transmission of the brain signals from the device 110 to the computer system may be achieved by wireless communication. Depending on the transmission protocol, this wireless communication may add latency, for example of the order of 50-200 ms for a Bluetooth protocol. The invention accounts for any such transmission delays by the calibration process. The calibration process accounts for any latency contribution to a phase shift between the modulated audio data and the brain response at the frequency of interest. The invention prevents any latency which is too large relative to the time period of the neural oscillation, and thus avoids deterioration of the efficacy of the control. It has been found that a 200 ms delay is acceptable when controlling theta oscillations. However a 200 ms delay would be unacceptable for controlling gamma oscillations with a typical time period of approximately 30 ms. It will be appreciated that there are alternative RF transmission protocols with low latencies time periods which would work in the case of gamma oscillations.

Figure 2 illustrates a workflow for brain wave modification for the user 100. The user's neural oscillations (brain waves) are modified responsive to measurements determined via electrical and/or magnetic sensors 140 located on and/or proximate to their scalp. A data file that comprises calibration information specific for the user, a calibration file 210, is provided to the device 110 by a connection 290. The connection 290 may be wired or wireless. Alternatively, the calibration file 210 may be stored on the device 110. The calibration file 210 comprises calibration data that is specific to the user 100. It will be understood that throughout the present specification, the phrase 'specific to the user' 100 means uniquely determined for a unique individual human being or a unique individual non-human animal. For example, the calibration file comprises data that encodes the neurodynamic response of an individual to a predetermined piece of music for a given hardware configuration. The phrase 'hardware configuration' means the specific arrangement of hardware that is involved in the workflow of measuring the neurodynamic of the user responsive to reproducing modified sound specifically adapted for the neurodynamics of the user. The calibration file also comprises data encoding the different positions the sensors 140 may be located on the head of the user 100.

Also provided to or stored on the device is a music file 220 that comprises digital instructions for generating music. The music file 220 is provided to the device by a connection 280. The connection 280 may be wired or wireless. Alternatively, the music file 220 may be stored locally on the device 110. The measurements of the neural oscillations of the user 100 are converted to analogue or digital signals by a transducer 200. These signals are then provided to the device 110 as a real-time input 240. The real-time input 240 may be provided via a wired or wireless connection. The transducer 200 is a hardware component that converts brain signals 250 of the user 100 that are measured by sensors, such as sensors 140, into an analogue or digital form that may be read and processed by the device 110. It is noted that in certain embodiments of the present invention, the transducer 200 may form part of the device 110 or the transducer 200 may be integrated into the device 110. In certain embodiments of the present invention, the transducer 200 may be separate from the device 110.

Responsive to the input received from the transducer 200 and the calibration file 210, the device 110 modifies the music file 220 via an algorithm. The modification to the music file 220 by the algorithm is such that upon playback to the user, the sound corresponding to the modified music field has an intended effect on the neural oscillations of a user. That is to say, the algorithm modifies certain elements of the music file, such that when sound corresponding to the modified music file is reproduced for the user 100, the neural oscillations of the user are modified in a desired fashion, such as amplification or suppression of brain oscillations of a certain frequency or within a certain frequency band. The modified music file is provided to an audio source via a real-time output 260. The real-time output 260 may be provided by a wired or wireless connection. The real-time output 260 is provided to an audio source 230. The audio source 230 is any hardware component that is capable of producing sound corresponding to a music file, such as headphones, earphones, speakers, and the like. The audio source 230 produces sound 270 corresponding to the real-time output 260. The sound 270 stimulates an auditory response in the user 100, which comprises a change in or maintenance of the neurodynamic state of the user 100. Responsive to the auditory stimulation induced by the sound 270, the user produces brain signals 250.

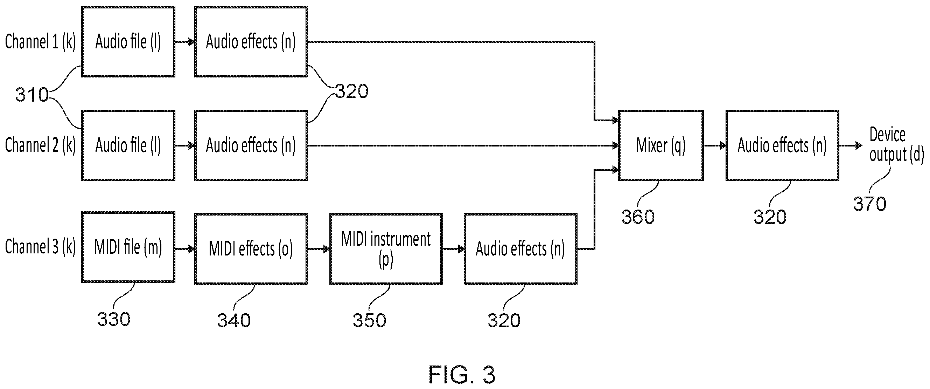

Figure 3 illustrates how channels of the music file 220 may be modified with audio effects 320. The music file 220 comprises a plurality of channels, each channel corresponding to one or more audio files 310 and/or one or more Musical Instrument Digital Interface (MIDI) files 330. It is noted that an audio file 310 may be understood to comprise a pre-recorded or pre-synthesised stream audio. It is also noted that a MIDI file 330 may be understood to comprise MIDI instructions that specify the timing, pitch, and volume of a sequence of notes. In accordance with the present invention, the audio files are modified with audio effects that are determined responsive to measured neural oscillations of the user. The audio effects alter characters of the audio such as volume, faders, filters, distortion, compression, pitch-shifting, time-warping, delay, stereo panning, reverb, echo, tremolo, and the like. In respect of a MIDI channel, MIDI effects 340 may be applied to a MIDI file 330 responsive to measured neural oscillations of the user. In addition, audio effects 320 may be applied to the output of a MIDI instrument. Multiple audio channels of the music file 220 are provided as inputs to a mixer 360 wherein the audio channels are combined. Further audio effects 320 may be applied to the mixed sound prior to the mixed sound being output from the mixer 360.

The invention may generate sound from MIDI instructions or from MIDI effects rather than generating from voice recordings. With MIDI data it is possible to shift notes forward or backwards in time. For example if it was desired to synchronise the sound of drum hits to the brain oscillation, this would be easy to achieve using a MIDI track.

Further audio effects may be applied to the music file 220 when mixing and producing multitrack music. The digital audio effects may be in the form of 'plugin' software formats. The digital audio effects may be transmitted or downloaded from remote servers and may be supplied by third-party sources.

The invention may use a plurality of predetermined audio effects for play-back. Alternatively the invention may receive a music creation file from a user, which enables the user to create a particular piece of brain-responsive music only.

The music file 220 may be created in digital audio workstation (DAW) software. The resultant music file 220 may be in a file-format that contains the instructions to generate the music in real-time in conjunction with brain signals.

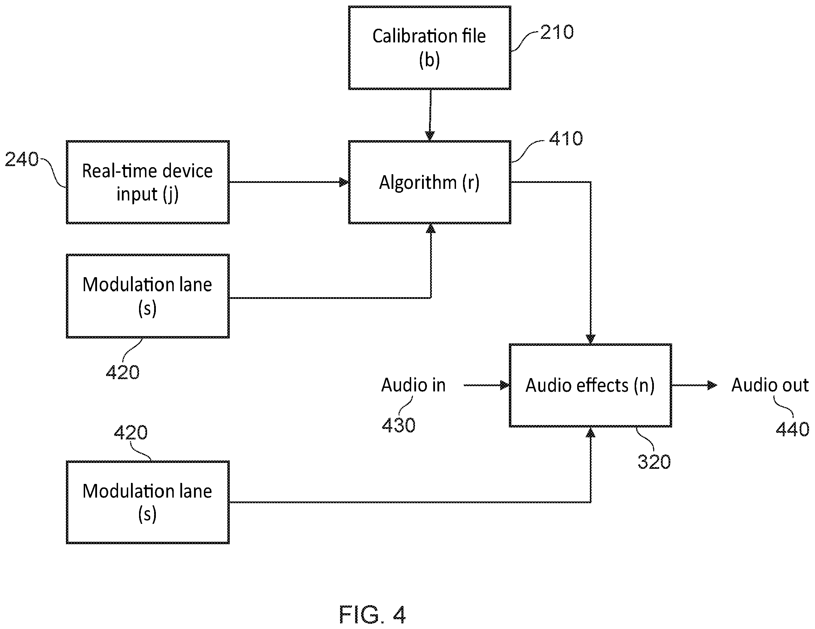

Figure 4 illustrates how the device 110 modifies the music file 220 using an algorithm 410. The device has access to the calibration file 210 and receives real-time input 240. The device 110 executes the algorithm 410 that receives as input, calibration data for the unique individual user and the specific hardware configuration being used. The algorithm 410 modifies channels of the music file 220 by applying audio effects 320 to an audio file 430. It will also be understood that the algorithm 410 may also apply MIDI effects to a MIDI file. The modified music file is then provided as output 440 to an audio source.

A modulation lane is a set of instructions that determine how a parameter, such as an audio effect, changes through the course of a piece of music. A modulation lane may include a sequence of values for that parameter sampled at some fixed rate. Alternatively a modulation lane may include information about when the parameter value changes and by how much. A modulation lane may be encoded as a sequence of MIDI control change messages.

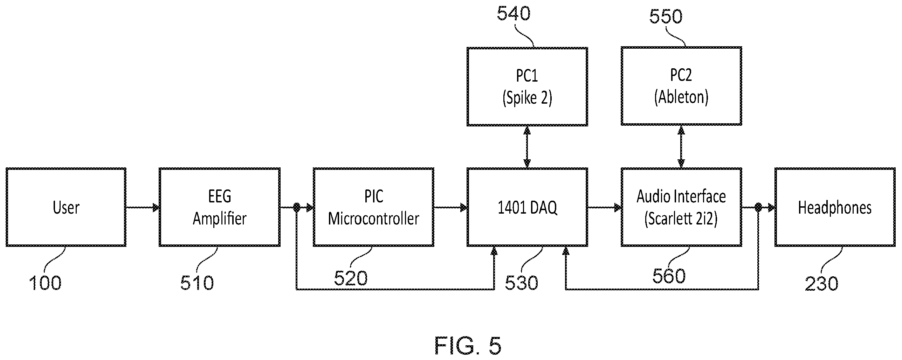

Figure 5 illustrates a workflow for how electroencephalography (EEG) may be used to provide sound to the user 100 via headphones 230. The sound may be modified in real-time responsive to measured brain waves of the user 100. EEG signals may be determined for the user 100 via electrodes positioned on the head of the user 100, for example at right mastoid and forehead locations. EEG signals are determined for the user 100, which are amplified by EEG amplifier 510. The EEG amplifier 510 may amplify the EEG signals detected for the user 100 by approximately 10,000 times. The amplified EEG signals are output to a microcontroller 520 that executes an algorithm.

The DAQ (data acquisition system) 530 and PC1 540 record brain signals via digital to analog converters to enable subsequent analysis of the data. The DAQ 530 also transmits the brain data via analog to digital converters to the audio interface. The audio interface converts the output back into digital form and sends it to PC2 550 which runs the audio software to generate the music. The invention includes this signal path of brain signal - digitised by DAQ 530 - output as analog signal to audio interface, to take into account that audio interfaces typically high-pass filter inputs because audio interfaces are configured to expect audio signals. The invention uses the DAQ 530 to arrange the brain signal on an audio frequency carrier wave to circumvent the filters. The audio interface is also used to output the music to the listeners headphones.

It will be appreciated that the arrangement illustrated in Fig. 5 is merely an example hardware to implement the invention. It is possible to use alternative configurations, for example with brain signals transmitted directly into the audio software via a Bluetooth link.

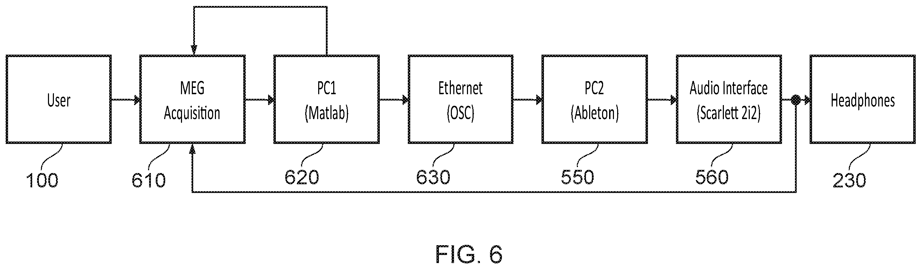

Figure 6 illustrates a workflow for how magnetoencephalography (MEG) may be used to provide sound to the user 100 via the headphones 230. The sound may be modified in real-time responsive to measured brain waves of a user.

The MEG 610 records the brain signals. PC1 620 applies processing to these brain signals, for example a phase-shift. The output is then transmitted to the audio software over an ethernet connection, rather than through the audio interface using a UDP protocol, for example Open Sound Control. In this case the audio interface is being used to output the music to the listener's headphones.

In this case the audio effect parameter being modulated is the cut-off frequency of a low-pass filter. Increasing this frequency allows more of the higher harmonics of the sound to pass through. Therefore it both increases the volume and changes the timbre of the sound. This is commonly described as a wah-wah effect.

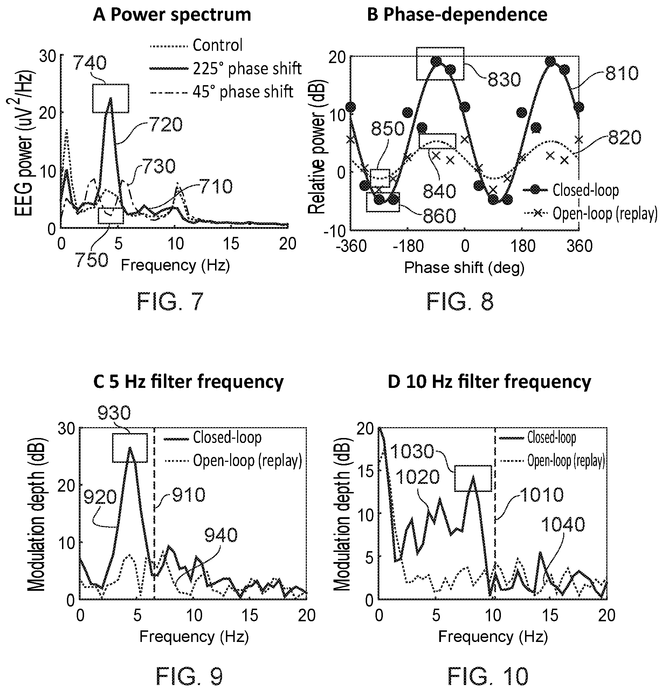

Figure 7 illustrates a graph of a power spectrum of EEG signals for frequencies up to 20 Hz. In this case the frequency of 5Hz is being targeted for modulation. EEG power is plotted against frequency for three cases: a control 710, a 225° phase-shift 720, and a 45° phase-shift 730. The phase-shifts are applied to the measured neural oscillations, which are used as inputs for an algorithm to apply audio effects to music that is played back to the user 100. As can be seen from the plot showing the 225° phase-shift 720, by introducing a 225° phase-shift, amplification of the neural oscillations with respect to the control 710 is achieved that peaks at peak 740. Conversely, suppression of the neural oscillations with respect to the control 710 is shown at the trough 750 of the 45° phase-shift 730. Figure 7 demonstrates the effectiveness of the present invention of amplifying/supressing neural oscillations at a particular frequency or within a particular frequency band. It will be understood that frequencies other than approximately 5Hz may be chosen for amplification/suppression. In this case 5Hz is the centre frequency of the FIR filter that phase-shifts the brain signal. The FIR filter is an alternative type of filter to the 'wah-wah' filter discussed in relation to Fig. 6.

The FIR filter allows selection of the frequency of brain oscillation to be controlled. Fig. 7 illustrates the effect of two different settings, 5 Hz (theta band) and 10 Hz (alpha band). In this case the FIR filter bandpass filters and phase-shifts the signal.

Figure 8 shows the relative power of neural oscillations of 5Hz with respect to a control for a closed-loop example and an open-loop example. As is shown for a curve for a closed-loop 810 and for a curve for an open-loop 820, both the closed-loop and open-loop achieve peak amplification, for example peaks 830, 840 at similar phase-shifts. Moreover, both the closed-loop and open-loop achieve maximal suppression, as shown by troughs 850, 860 at similar phase-shifts. However, the extent to which the neural oscillations are amplified/suppressed is greater with respect to the control in the closed-loop experiment than in the open-loop experiment. Figure 8 demonstrates the superiority of a close-loop system.

Figure 9 illustrates the modulation depth against frequency of a closed-loop signal 920 similar to as featured in Figure 7 and an open-loop signal 930. The modulation depth is measured from the sinusoidal fit of power vs. phase-shift. The modulation depth is the peak-to-peak amplitude of the fitted sine wave, expressed as a log power ratio (dB). The power of the brain signal is modulated. All phase-shifts between 0-360 degrees are applied in 45 degree steps. The phase-shifts are plotted on the horizontal axis of Figure 8.

Figure 10 is a similar arrangement to Figure 9. In Figure 10, the frequency of the filter is set to 10 Hz. In Figure 10, the modulation occurs at a higher frequency.

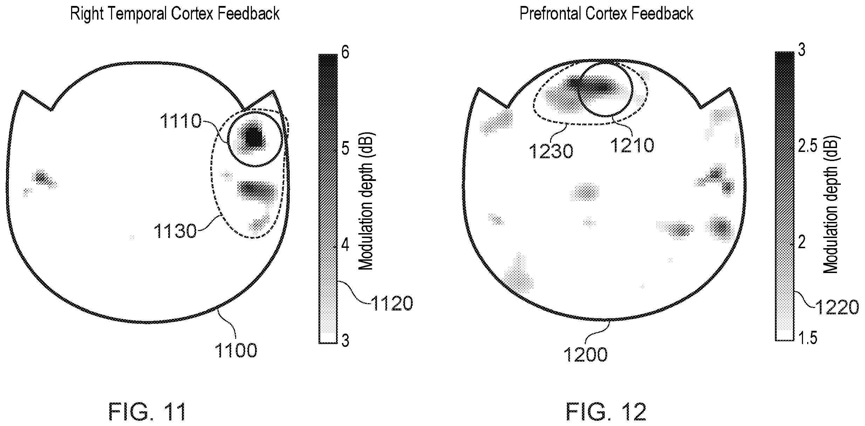

Figures 11 and 12 illustrate a magnetoencephalogram of the right temporal cortex 1100 of a person and prefrontal cortex of a person 1200, respectively. Respective regions of activity 1130, 1230 are shown. In each figure, the neural oscillations of the respective parts of the user's brain are measured responsive to musical playback. These measurements are then used in accordance with the workflow of Figure 2 such that the music is modified in real time responsive to the user's contemporaneous brain activity. The depth of shading within the cortexes 1100, 1200 shown indicates the modulation depth 1120, 1220 of respective neural oscillations measured. Figures 11 and 12 show that the modified music then induces a response in the neural oscillations of the brain at the locations of the brain that are being observed to modify the music. In Figure 11, a location in the right temporal cortex of a user of neural oscillation measurement 1110 coincides with induced neural activity at or adjacent to the same location. In Figure 12, a location in the prefrontal cortex of a user of neural oscillation measurement 1210 coincides with induced neural activity at or adjacent to the same location.

The modulation depth information indicates that there is a phase-dependent modulation of the neural oscillation. The modulation depth information shows frequencies and/or brain regions where the neural oscillation may be either boosted or suppressed by tuning the phase to produce positive or negative feedback.

As part of the cross-spectral analysis, the cross-spectrum between two signals is calculated from the Fourier transform of the two signals. At each frequency, the Fourier coefficient of the first signal is multiplied by the complex conjugate of the Fourier coefficient of the second signal. The phase of the cross-spectrum may be analysed to determine the phase relationship between the two signals.

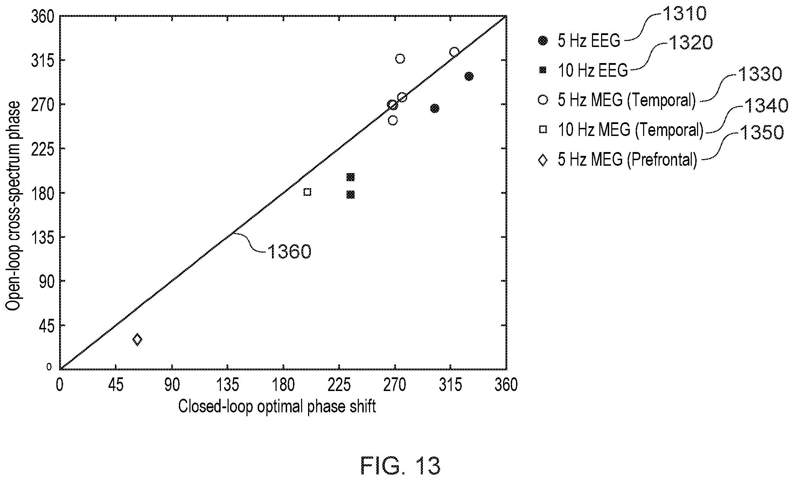

Figure 13 shows a scatter plot of the optimal phase shift for a closed-loop system against the optimal phase shift for an open-loop system for different filters, for different measurement methods (i.e. EEG or MEG), and for different measurement locations (e.g. temporal cortex or prefrontal cortex). Figure 13 shows the benefit of calibration of the phase shift as the optimal phase shift is not identical for each filter frequency and measurement method. Moreover, the Figure 13 also shows that the optimal phase-shifts for a closed-loop system are similar to the optimal phase-shifts for an open-loop system. Thus an open-loop system may be used to calibrated a closed-loop system.

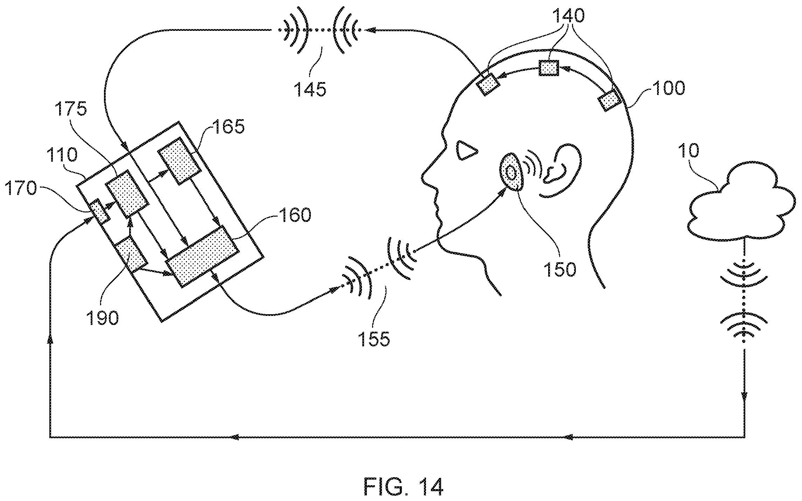

Referring to Figs. 14 to 17 there is illustrated the system according to the invention for closed-loop modulation of audio data, which is similar to that described previously in relation to Fig. 2, and similar elements in Figs. 14 to 17 are assigned the same reference numerals.

In this case the system is suitable for use with a smartphone device or tablet device or mobile communication device (Fig. 14). There may be for a wired or wireless connection 145 of the sensors 140 to a processing device 110. There may be a wireless or wired connection 155 of the processing device 110 to the loudspeaker/headphones 150. The historical brain signals are stored in a database 165. The music files database 175 on the processing device 110 may contain complete music files or may be used for buffering music streamed from a remote source 10.

The user may control the processing device 110 to select the song from the music files database 175 or from the remote source 10. The user may control the processing device 110 to select the filter to be applied by the processing element 160, and to select which frequency to be modulated as well as whether the modulation is to be boosting or suppressing. The user control may also be used to select filters with different listening experiences for the same frequency and effect.

In a streaming situation the remote source 10 acts as the music database and may contain newly created music or the user's previously created or previously obtained music files. The music may be downloaded or streamed from the remote database 10 to the processing device 110 where it is processed by the processing element 160 together with the user's brain signals.

In this case the system includes a user interface element 190 to facilitate user control. The user 100 may use the user interface element 190 to select the song from the music files database 175 or from the remote source 10. The user 100 may use the user interface element 190 to select the filter to be applied by the processing unit 160. The filter selects the frequency to be modulated, and whether the modulation is to be boosting or suppressing. The user control element 190 may be used to select filters with different listening experiences for the same frequency and effect.

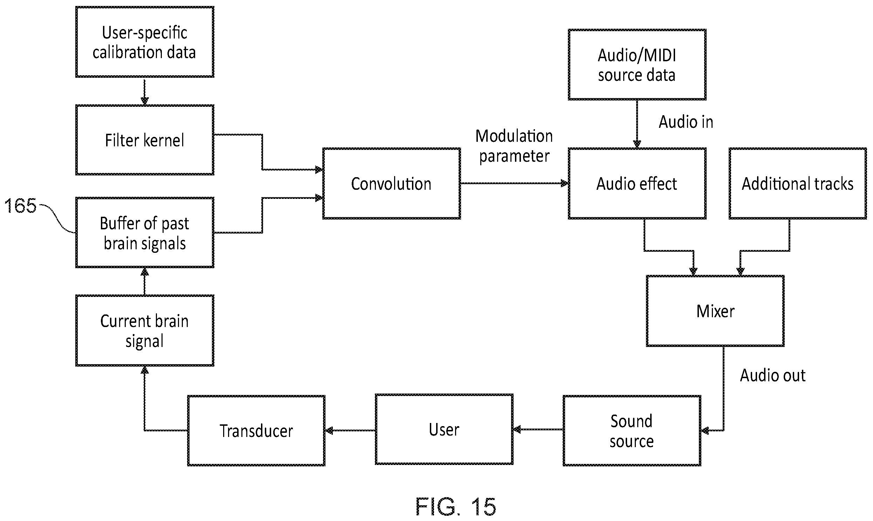

In this case the system includes a buffer or database 165 for storing the past history of brain signals (Fig. 15).

During closed-loop operation (Fig. 16), the open-loop transfer function is augmented with the feedback policy H(w). The feedback system thus has a closed-loop transfer function G'(w) which depends on the product G(w)H(w). The closed-loop transfer function is maximised if G(w)H(w) is real and positive which is obtained by setting phi=-arg(G(w)) and A as real positive. This corresponds to positive feedback which amplifies the brain signals at frequency w. By contrast, the closed-loop transfer function is minimized if G(w)H(w) is real and negative which is obtained by setting phi=-arg(G(w)) and A as real negative. This corresponds to negative feedback which attenuates brain signals at frequency w.

During open-loop calibration (Fig. 17), the open-loop transfer function G(w) is characterised between modulated music and resultant brain signal at the frequency of interest (w). Therefore the music is modulated with a predetermined pattern, e.g. white noise, x(t) and the resultant brain signal y(t) is measured. By converting these signals into the frequency domain by calculating the Fourier coefficients, the phase response arg(G(w)) is calculated from the phase of the cross-spectrum between x and y.

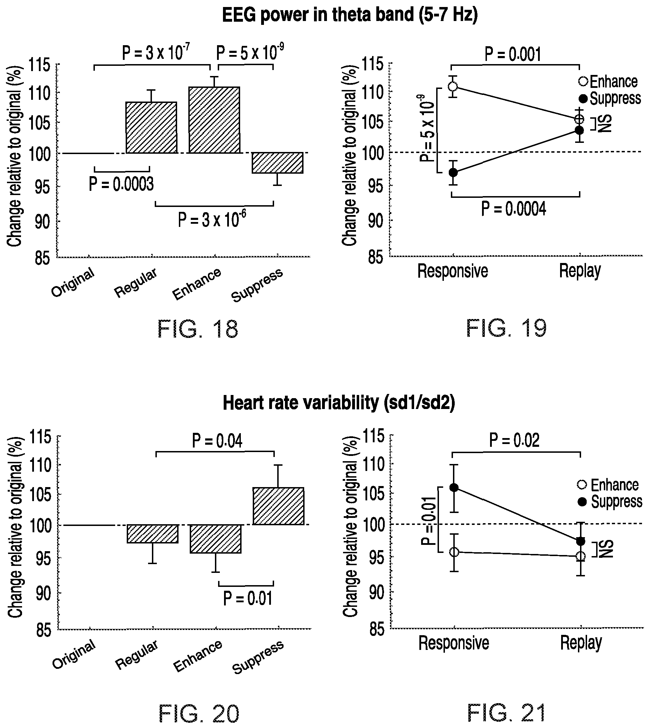

Figs. 18 and 19 illustrate a first example of the system according to the invention in use.

In this first example the system according to the invention was used with a sample of 40 subject users. The initial audio data delivered to each subject user was a 30 second piece of piano music. The differential EEG power between the forehead (AFz) and the mastoid of each subject user was measured.

Figs. 18 and 19 illustrate the results of this first example under different conditions with and without applying the method of the invention to modulate the theta brainwaves of the subject users.

Fig. 18 illustrates the average changes in EEG power relative to the original, unaltered music. 'Regular' refers to when the music is altered in a similar way at a regular frequency but is not synchronized with the brain activity of the subject users. 'Enhance' refers to when the music is altered so as to increase the amplitude of theta oscillations. 'Suppress' refers to when the music is altered so as to reduce the amplitude of theta oscillations.

Fig. 19 illustrates a comparison of the difference in theta power between enhance and suppress conditions. 'Responsive' refers to conditions in which the music is altered in real-time based on brain activity. 'Replay' refers to conditions in which the same music is played a second time. Because the second time the music is played, the music is no longer synchronised with the listener's brain, these conditions act as a control to demonstrate that the modulation of brain activity is due to the closed-loop feedback between the brain and the music, rather than a direct effect of the music alone.

Figs. 20 and 21 illustrate another physiological parameter, in this case heart rate variability, which changed when the theta brainwaves of the subject users were supressed. The data in Figs. 20 and 21 illustrate average heart rate variability. The SD1/SD2 ratio reflects the balance between parasympathetic and sympathetic tone that is derived from the ratio of high-frequency and low-frequency heart rate variability. Higher values of this heart rate variability measure are associated with relaxation, and lower values of heart rate variability are associated with emotional arousal. Reduced heart rate variability may be causally related to increased cardiovascular disease in anxiety disorders and may also be a correlate of autonomic responses to emotionally positive music. These data demonstrate that the condition in which theta power was reduced by the music was also associated with an increase in the SD1/SD2 heart rate variability measure.

Throughout this patent specification reference is made to "sound". It will be understood that sound is a vibration that propagates as an acoustic wave through a transmission medium such as air, gas, liquid or solid. An acoustic wave is energy propagating through a medium by means of compression and decompression of the medium. An acoustic wave has a frequency in the range 20Hz to 20kHz.

Throughout this patent specification reference is made to "surgery". It will be understood that surgery refers to an invasive step representing a substantial physical intervention on the body which requires professional medical expertise to be carried out and which entails a health risk even when carried out with the required professional care and expertise. Aptly surgery comprises an invasive step.

Throughout this patent specification reference is made to "therapy". It will be understood that therapy refers to the curing of a disease or malfunction of the body and covers prophylactic treatment. In particular, therapy is concerned with bringing a body from a pathological state back to its normal healthy state, and with preventing a pathological state. Aptly a therapeutic effect occurs only in a subject in a pathological state. Aptly a therapeutic effect does not occur in a healthy person.

Throughout this patent specification reference is made to "healthy". It will be understood that health refers to person in a good physical or mental condition not displaying clinical signs of disease, infection or illness.

Throughout this patent specification reference is made to "non-therapeutic". It will be understood that non-therapeutic refers to an effect not relating to therapy. Aptly a non-therapeutic effect occurs in a normal, healthy subject.

Throughout this patent specification reference is made to "clinical signs". It will be understood that clinical signs refer to objective terms for describing abnormalities that are detected by a clinician during a clinical observation or examination, for example vital signs or blood test results. Aptly clinical signs are objective evidence of disease, these are clearly different to a symptom, which is a subjective manifestation of disease apparent to the patient themselves, for example a headache.

Throughout this patent specification reference is made to "pathological state" It will be understood that pathological state refers to a physical condition that is caused by disease.

Reference is also made throughout this patent specification to a "transducer". A transducer is a device that converts energy from one form to another. When a physical quantity is converted to a mechanical quantity the transducer may be referred to as a mechanical transducer. When a physical quantity is converted to an electrical quantity the transducer may be referred to as an electrical transducer. A microphone is an example of an electrical transducer in the sense that a microphone converts sound into an electrical signal.

Reference is also made throughout this patent specification to an "audio signal". An audio signal is a representation of sound. For example, a changing level of voltage for analogue signals or a series of binary numbers for digital signals. Audio signals have associated frequencies in the audio frequency range of 20Hz to 20kHz.

Reference is also made throughout this patent specification to a "modulatable effect". A modulatable effect may be a volume or amplitude or pitch or timbre or attack or decay of tone. The effect is modulatable in the sense that it can be varied. For example a modulatable effect may be varied by selectively varying a respective modulation parameter. The modulatable effect may be a parameter that effects the volume of the audio signal, such as a gain knob. The modulatable effect may be pitch of the audio signal, such as a pitch shifting effect. The modulatable effect may be timbre, such as filter, saturation, or distortion. The modulatable effect may be timing of the audio signal, such as delays. The modulatable effect may be stereo width of the audio signal, such as mid/side balance.

Certain embodiments of the present invention provide a platform to enable the production and distribution of "closed-loop" music to selectively boost and/or supress brain oscillations in desired frequency bands in desired brain areas of a target user. The target user may be a human or animal. Certain non-medical non-therapeutic applications are mindfulness, meditation, memory enhancement and healthy sleep. Alternatively certain embodiments of the present invention can provide medical therapeutic applications, for example improving mental health or reducing the effect of dementia related disease.

Still further embodiments of the present invention provide an application for providing musical opportunities to composers. These applications enable a personalised musical experience for a target user such as a listener listening to music via a loudspeaker or headphones. This increases the creative possibilities for composers relative to prior art techniques.

Certain embodiments of the present invention provide a Virtual Studio Technology (VST) plugin to allow production of closed-loop music inside commercial Digital Audio Workstations (DAWs).

Still other embodiments of the present invention provide a mobile app for real-time processing and playing of closed-loop music to selectively modify a neural oscillation pattern in a brain of a target user.

Certain embodiments of the present invention provide a cloud platform for sharing and distributing music. Optionally this may include a user accessing the platform via a subscription service.

Throughout the description and claims of this specification, the words "comprise" and "contain" and variations of them mean "including but not limited to" and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of the features and/or steps are mutually exclusive. The invention is not restricted to any details of any foregoing embodiments. The invention extends to any novel one, or novel combination, of the features disclosed in this specification (including any accompanying claims, abstract and drawings), or to any novel one, or any novel combination, of the steps of any method or process so disclosed.

## Claims

1. A method of closed-loop modulation of audio data, the method comprising the steps of:
receiving first initial audio data,
receiving a first set of neural oscillation data for a first time period,
obtaining one or more historical neural oscillation data for one or more historical time periods previous to the first time period,
determining first modulated audio data based on the first initial audio data, the first set of neural oscillation data, the one or more historical neural oscillation data,
and a configured input-output relationship, and
delivering the first modulated audio data to a user device,
**characterised in that** the method further comprises a step of determining the configured input-output relationship, wherein the configured input-output relationship is determined by open-loop calibration of a neural oscillation modulator. wherein the method comprises the steps of:
receiving calibration audio data,
delivering the calibration audio data to a user device,
responsive to the calibration audio data delivered to the user device, receiving calibration neural oscillation data for the user,
determining a phase shift between the calibration audio data and the calibration neural oscillation data, and
the configured input-output relationship is determined based on the phase shift.

2. A method as claimed in claim 1 wherein the method comprises the steps of:
receiving second initial audio data,
receiving a second set of neural oscillation data for a second time period subsequent to the first time period,
obtaining one or more historical neural oscillation data for one or more historical time periods previous to the second time period,
determining second modulated audio data based on the second initial audio data, the second set of neural oscillation data, the one or more historical neural oscillation data, and the configured input-output relationship, and
delivering the second modulated audio data to the user device.

3. A method as claimed in any of claims 1 to 2 wherein the audio data comprises music data, the music data comprising in particular at least one of MIDI instruction data, MIDI effects data, human voice recording data, and instrumental recording data, pre-generated music data.

4. A method as claimed in claim 3 wherein the music data comprises two or more tones, and/or voices, and/or acoustic structures, and the music data varies over time in a pre-determined manner.

5. A method as claimed in any of claims 1 to 4 wherein the first set of neural oscillation data for the first time period is received from one or more sensors, in particular from one or more Electroencephalography sensors mountable to a scalp of the user.

6. A method as claimed in claim 5 wherein the method comprises the step of locating the one or more sensors at one or more desired areas of the head of the user to target neural oscillation activity at one or more corresponding desired areas of the brain of the user.

7. A method as claimed in any of claims 1 to 6 wherein the first set of neural oscillation data comprises cortical activity.

8. A method as claimed in any of claims 1 to 7 wherein the first set of neural oscillation data is phase shifted based on the one or more historical neural oscillation data and the configured input-output relationship to determine the first modulated audio data.

9. A method as claimed in any of claims 1 to 8 wherein the first set of neural oscillation data, and the one or more historical neural oscillation data are filtered to determine the first modulated audio data, wherein the first set of neural oscillation data, and the one or more historical neural oscillation data are bandpass filtered to determine the first modulated audio datain particular using a finite impulse response filter to determine the first modulated audio data, the finite impulse response filter having in particular an impulse response of the form: $h \left(t\right) = A cos \left(ωt+φ\right) {e}^{- k ω t} - C$ where
*h*(*t*) is the impulse response of the filter,
*A* is a real number that sets the closed loop feedback gain,
*C* is a real number chosen such that the DC gain is zero,
ω is the angular frequency of the neural oscillation to be affected,
*t* is time,
*k* is a positive real number that determines band width of the filter and a phase shift of each filter,
φ is the phase-shift of the input-output relationship.

10. A method as claimed in any of claims 1 to 9 wherein the method comprises the steps of:
receiving two or more sets of neural oscillation data for the first time period, and
the two or more sets of neural oscillation data, and the one or more historical neural oscillation data are filtered using two or more filters to determine the first modulated audio data with a plurality of audio effects,
each filter being configured to target a different frequency range.

11. A method as claimed in any of claim 10 wherein the method comprises the step of configuring a filter of the neural oscillation modulator based on the configured input-output relationship.

12. A method as claimed in any of claims 10 to 11 wherein the phase shift between the calibration audio data and the calibration neural oscillation data is determined at the one or more selected frequency ranges.

13. The method as claimed in any of claims 1 to 12, further comprising:
providing a respective pre-recorded or pre-synthesised stream of audio data via each channel source or providing a respective stream of MIDI instructions that specify at least one predetermined characteristic for a sequence of notes via each channel source.

14. A system for closed-loop modulation of audio data, the system comprising:
a neural oscillation modulator for receiving first initial audio data, receiving a first set of neural oscillation data for a first time period, and obtaining one or more historical neural oscillation data for one or more historical time periods previous to the first time period,
the neural oscillation modulator being configured to determine first modulated audio data based on the first initial audio data, the first set of neural oscillation data, the one or more historical neural oscillation data, and a configured input-output relationship,
the neural oscillation modulator being configured to deliver the first modulated audio data to a user device, in particular to a portable consumer device, wherein the system comprises means configured to perform the method of any of claims 1 to 13.

15. A computer program product comprising computer program code capable of causing a computer system to perform a method as claimed in any of claims 1 to 13 when the computer program product is run on a computer system.

## Patentansprüche

1. Verfahren für eine Modulation mit geschlossenem Regelkreis von Audiodaten, das Verfahren umfassend die Schritte:
Empfangen erster anfänglicher Audiodaten,
Empfangen eines ersten Satzes von Daten einer neuronalen Oszillation für eine erste Zeitspanne,
Erhalten von ein oder mehreren historischen Daten der neuronalen Oszillation für eine oder mehrere historische Zeitspannen vor der ersten Zeitspanne,
Bestimmen erster modulierter Audiodaten basierend auf den ersten anfänglichen Audiodaten, dem ersten Satz von Daten der neuronalen Oszillation, dem einen oder den mehreren historischen Daten der neuronalen Oszillation und einer konfigurierten Eingabe-Ausgabe-Beziehung, und
Liefern der ersten modulierten Audiodaten an eine Benutzervorrichtung,
**dadurch gekennzeichnet, dass** das Verfahren ferner einen Schritt zum Bestimmen der konfigurierten Eingabe-Ausgabe-Beziehung umfasst, wobei die konfigurierte Eingabe-Ausgabe-Beziehung durch eine Kalibrierung mit offenem Regelkreis eines Modulators der neuronalen Oszillation bestimmt wird, wobei das Verfahren die Schritte umfasst:
Empfangen von Kalibrierungsaudiodaten,
Liefern der Kalibrierungsaudiodaten an eine Benutzervorrichtung,
als Reaktion auf die an die Benutzervorrichtung gelieferten Kalibrierungsaudiodaten, Empfangen von Kalibrierungsdaten der neuronalen Oszillation für den Benutzer,
Bestimmen einer Phasenverschiebung zwischen den Kalibrierungsaudiodaten und den Kalibrierungsdaten der neuronalen Oszillation, und
die konfigurierte Eingabe-Ausgabe-Beziehung wird basierend auf der Phasenverschiebung bestimmt.

2. Verfahren nach Anspruch 1, wobei das Verfahren die Schritte umfasst:
Empfangen zweiter anfänglicher Audiodaten,
Empfangen eines zweiten Satzes von Daten der neuronalen Oszillation für eine zweite Zeitspanne nach der ersten Zeitspanne,
Erhalten eines oder mehrerer historischer Daten der neuronalen Oszillation für eine oder mehrere historische Zeitspannen vor der zweiten Zeitspanne,
Bestimmen zweiter modulierter Audiodaten basierend auf den zweiten anfänglichen Audiodaten, dem zweiten Satz von Daten der neuronalen Oszillation, dem einen oder den mehreren historischen Daten der neuronalen Oszillation und der konfigurierten Eingabe-Ausgabe-Beziehung, und
Liefern der zweiten modulierten Audiodaten an die Benutzervorrichtung.

3. Verfahren nach einem der Ansprüche 1 bis 2, wobei die Audiodaten Musikdaten umfassen, die Musikdaten umfassend insbesondere mindestens eines von MIDI-Befehlsdaten, MIDI-Effektdaten, Aufzeichnungsdaten menschlicher Stimme und Instrumentalaufzeichnungsdaten, vorgenerierten Musikdaten.

4. Verfahren nach Anspruch 3, wobei die Musikdaten zwei oder mehr Töne und/oder Stimmen und/oder akustische Strukturen umfassen, und die Musikdaten im Laufe der Zeit in einer vorbestimmten Weise variieren.

5. Verfahren nach einem der Ansprüche 1 bis 4, wobei der erste Satz von Daten der neuronalen Oszillation für die erste Zeitspanne von einem oder mehreren Sensoren empfangen wird, insbesondere von einem oder mehreren Elektroenzephalographiesensoren, die an einer Kopfhaut des Benutzers anbringbar sind.

6. Verfahren nach Anspruch 5, wobei das Verfahren den Schritt zum Anordnen des einen oder der mehreren Sensoren an einem oder mehreren gewünschten Bereichen des Kopfes des Benutzers umfasst, um auf eine Aktivität der neuronalen Oszillation an einem oder mehreren entsprechenden gewünschten Bereichen des Gehirns des Benutzers abzuzielen.

7. Verfahren nach einem der Ansprüche 1 bis 6, wobei der erste Satz von Daten der neuronalen Oszillation kortikale Aktivität umfasst.

8. Verfahren nach einem der Ansprüche 1 bis 7, wobei der erste Satz von Daten der neuronalen Oszillation basierend auf dem einen oder den mehreren historischen Daten der neuronalen Oszillation und der konfigurierten Eingabe-Ausgabe-Beziehung phasenverschoben wird, um die ersten modulierten Audiodaten zu bestimmen.

9. Verfahren nach einem der Ansprüche 1 bis 8, wobei der erste Satz von Daten der neuronalen Oszillation und das eine oder die mehreren historischen Daten der neuronalen Oszillation gefiltert werden, um die ersten modulierten Audiodaten zu bestimmen, wobei der erste Satz von Daten der neuronalen Oszillation und das eine oder die mehreren historischen Daten der neuronalen Oszillation bandpassgefiltert werden, um die ersten modulierten Audiodaten zu bestimmen, insbesondere unter Verwendung eines Filters mit endlicher Impulsantwort, um die ersten modulierten Audiodaten zu bestimmen, wobei das Filter mit endlicher Impulsantwort insbesondere eine Impulsantwort der Form aufweist: $h \left(t\right) = A cos \left(ωt+φ\right) {e}^{- kωt} - C$ wobei:
*h(t)* die Impulsantwort des Filters ist,
*A* eine reelle Zahl ist, die die Rückkopplungsverstärkung mit geschlossenem Regelkreis festlegt,
*C* eine reelle Zahl ist, die derart gewählt ist, dass die Gleichstromverstärkung null ist,
ω die Kreisfrequenz der zu beeinflussenden neuronalen Oszillation ist,
*t* die Zeit ist,
*k* eine positive reelle Zahl ist, die die Bandbreite des Filters und eine Phasenverschiebung jedes Filters bestimmt,
φ die Phasenverschiebung der Eingabe-Ausgabe-Beziehung ist.

10. Verfahren nach einem der Ansprüche 1 bis 9, wobei das Verfahren die Schritte umfasst:
Empfangen von zwei oder mehr Sätzen von Daten der neuronalen Oszillation für die erste Zeitspanne, und
die zwei oder mehr Sätze von Daten der neuronalen Oszillation und das eine oder die mehreren historischen Daten der neuronalen Oszillation werden unter Verwendung von zwei oder mehr Filtern gefiltert, um die ersten modulierten Audiodaten mit einer Vielzahl von Audioeffekten zu bestimmen,
wobei jedes Filter konfiguriert ist, um auf einen anderen Frequenzbereich abzuzielen.

11. Verfahren nach einem von Anspruch 10, wobei das Verfahren den Schritt zum Konfigurieren eines Filters des Modulators der neuronalen Oszillation basierend auf der konfigurierten Eingabe-Ausgabe-Beziehung umfasst.

12. Verfahren nach einem der Ansprüche 10 bis 11, wobei die Phasenverschiebung zwischen den Kalibrierungsaudiodaten und den Kalibrierungsdaten der neuronalen Oszillation in dem einen oder den mehreren ausgewählten Frequenzbereichen bestimmt wird.

13. Verfahren nach einem der Ansprüche 1 bis 12, ferner umfassend:
Bereitstellen eines jeweiligen voraufgezeichneten oder vorsynthetisierten Audiodatenstroms über jede Kanalquelle oder Bereitstellen eines jeweiligen Stroms von MIDI-Anweisungen, die mindestens eine vorbestimmte Eigenschaft für eine Notensequenz spezifizieren, über jede Kanalquelle.

14. System für die Modulation mit geschlossenem Regelkreis von Audiodaten, das System umfassend:
einen Modulator der neuronalen Oszillation zum Empfangen erster anfänglicher Audiodaten, Empfangen eines ersten Satzes von Daten der neuronalen Oszillation für eine erste Zeitspanne und Erhalten von einem oder mehreren historischen Daten der neuronalen Oszillation für eine oder mehrere historische Zeitspannen vor der ersten Zeitspanne, wobei der Modulator der neuronalen Oszillation konfiguriert ist, um erste modulierte Audiodaten basierend auf den ersten anfänglichen Audiodaten, dem ersten Satz von Daten der neuronalen Oszillation, dem einen oder den mehreren historischen Daten der neuronalen Oszillation und einer konfigurierten Eingabe-Ausgabe-Beziehung zu bestimmen, wobei der Modulator der neuronalen Oszillation konfiguriert ist, um die ersten modulierten Audiodaten an eine Benutzervorrichtung, insbesondere an eine tragbare Verbrauchervorrichtung, zu liefern, wobei das System Mittel umfasst, die konfiguriert sind, um das Verfahren nach einem der Ansprüche 1 bis 13 durchzuführen.

15. Computerprogrammprodukt, umfassend Computerprogrammcode, der geeignet ist, ein Computersystem zu veranlassen, ein Verfahren nach einem der Ansprüche 1 bis 13 durchzuführen, wenn das Computerprogrammprodukt auf einem Computersystem ausgeführt wird.

## Revendications

1. Procédé de modulation en boucle fermée de données audio, le procédé comprenant les étapes consistant à :
recevoir des premières données audio initiales,
recevoir un premier ensemble de données d'oscillation neuronale pour un premier laps de temps,
obtenir une ou plusieurs données d'oscillation neuronale historiques pour un ou plusieurs laps de temps historiques précédant le premier laps de temps,
déterminer des premières données audio modulées en fonction des premières données audio initiales, du premier ensemble de données d'oscillation neuronale, de la ou des données d'oscillation neuronale historiques, et d'une relation d'entrée-sortie configurée, et
délivrer les premières données audio modulées à un dispositif utilisateur,
**caractérisé en ce que** le procédé comprend en outre une étape consistant à déterminer la relation d'entrée-sortie configurée, dans lequel la relation d'entrée-sortie configurée est déterminée par étalonnage en boucle ouverte d'un modulateur d'oscillation neuronale, dans lequel le procédé comprend les étapes consistant à :
recevoir des données audio d'étalonnage,
délivrer les données audio d'étalonnage à un dispositif utilisateur,
en réponse aux données audio d'étalonnage délivrées au dispositif utilisateur, recevoir des données d'oscillation neuronale d'étalonnage pour l'utilisateur,
déterminer un déphasage entre les données audio d'étalonnage et les données d'oscillation neuronale d'étalonnage, et
la relation d'entrée-sortie configurée est déterminée en fonction du déphasage.

2. Procédé selon la revendication 1 dans lequel le procédé comprend les étapes consistant à :
recevoir des secondes données audio initiales,
recevoir un second ensemble de données d'oscillation neuronale pour un second laps de temps suivant le premier laps de temps,
obtenir une ou plusieurs données d'oscillation neuronale historiques pour un ou plusieurs laps de temps historiques précédant le second laps de temps,
déterminer des secondes données audio modulées en fonction des secondes données audio initiales, du second ensemble de données d'oscillation neuronale, de la ou des données d'oscillation neuronale historiques, et de la relation d'entrée-sortie configurée, et
délivrer les secondes données audio modulées au dispositif utilisateur.

3. Procédé selon l'une quelconque des revendications 1 à 2 dans lequel les données audio comprennent des données musicales, les données musicales comprenant en particulier au moins l'une parmi des données d'instruction MIDI, des données d'effets MIDI, des données d'enregistrement vocal humain, et des données d'enregistrement instrumental, des données musicales prégénérées.

4. Procédé selon la revendication 3 dans lequel les données musicales comprennent au moins deux tonalités, et/ou voix, et/ou structures acoustiques, et les données musicales varient au fil du temps d'une manière prédéterminée.

5. Procédé selon l'une quelconque des revendications 1 à 4 dans lequel le premier ensemble de données d'oscillation neuronale pour le premier laps de temps est reçu à partir d'un ou plusieurs capteurs, en particulier à partir d'un ou plusieurs capteurs d'électroencéphalographie pouvant être montés sur un cuir chevelu de l'utilisateur.

6. Procédé selon la revendication 5 dans lequel le procédé comprend l'étape consistant à localiser le ou les capteurs au niveau d'une ou plusieurs zones souhaitées de la tête de l'utilisateur pour cibler une activité d'oscillation neuronale au niveau d'une ou plusieurs zones souhaitées correspondantes du cerveau de l'utilisateur.

7. Procédé selon l'une quelconque des revendications 1 à 6 dans lequel le premier ensemble de données d'oscillation neuronale comprend une activité corticale.

8. Procédé selon l'une quelconque des revendications 1 à 7 dans lequel le premier ensemble de données d'oscillation neuronale est déphasé en fonction de la ou des données d'oscillation neuronale historiques et de la relation d'entrée-sortie configurée pour déterminer les premières données audio modulées.

9. Procédé selon l'une quelconque des revendications 1 à 8 dans lequel le premier ensemble de données d'oscillation neuronale, et la ou les données d'oscillation neuronale historiques sont filtrés pour déterminer les premières données audio modulées, dans lequel le premier ensemble de données d'oscillation neuronale, et la ou les données d'oscillation neuronale historiques sont filtrés par passe-bande pour déterminer les premières données audio modulées en particulier à l'aide d'un filtre à réponse impulsionnelle finie pour déterminer les premières données audio modulées, le filtre à réponse impulsionnelle finie ayant en particulier une réponse impulsionnelle sous la forme : $h \left(t\right) = A cos \left(ωt+φ\right) {e}^{- kωt} - C$ où
*h*(*t*) est la réponse impulsionnelle du filtre,
*A* est un nombre réel qui définit le gain de rétroaction en boucle fermée,
*C* est un nombre réel choisi de telle sorte que le gain CC est nul,
*ω* est la fréquence angulaire de l'oscillation neuronale à affecter,
*t* est le temps,
*k* est un nombre réel positif qui détermine une largeur de bande du filtre et un déphasage de chaque filtre,
*φ* est le déphasage de la relation d'entrée-sortie.

10. Procédé selon l'une quelconque des revendications 1 à 9 dans lequel le procédé comprend les étapes consistant à :
recevoir au moins deux ensembles de données d'oscillation neuronale pour le premier laps de temps, et
les au moins deux ensembles de données d'oscillation neuronale, et la ou les données d'oscillation neuronale historiques sont filtrés à l'aide d'au moins deux filtres pour déterminer les premières données audio modulées avec une pluralité d'effets audio,
chaque filtre étant configuré pour cibler une plage de fréquences différente.

11. Procédé selon l'une quelconque de la revendication 10 dans lequel le procédé comprend l'étape consistant à configurer un filtre du modulateur d'oscillation neuronale en fonction de la relation d'entrée-sortie configurée.

12. Procédé selon l'une quelconque des revendications 10 à 11 dans lequel le déphasage entre les données audio d'étalonnage et les données d'oscillation neuronale d'étalonnage est déterminé au niveau de la ou des plages de fréquences sélectionnées.

13. Procédé selon l'une quelconque des revendications 1 à 12, comprenant en outre :
la fourniture d'un flux respectif préenregistré ou présynthétisé de données audio par l'intermédiaire de chaque source de canal ou la fourniture d'un flux respectif d'instructions MIDI qui spécifient au moins une caractéristique prédéterminée pour une séquence de notes par l'intermédiaire de chaque source de canal.

14. Système de modulation en boucle fermée de données audio, le système comprenant :
un modulateur d'oscillation neuronale permettant de recevoir des premières données audio initiales, de recevoir un premier ensemble de données d'oscillation neuronale pour un premier laps de temps, et d'obtenir une ou plusieurs données d'oscillation neuronale historiques pour un ou plusieurs laps de temps historiques précédant le premier laps de temps, le modulateur d'oscillation neuronale étant configuré pour déterminer des premières données audio modulées en fonction des premières données audio initiales, du premier ensemble de données d'oscillation neuronale, de la ou des données d'oscillation neuronale historiques, et d'une relation d'entrée-sortie configurée, le modulateur d'oscillation neuronale étant configuré pour délivrer les premières données audio modulées à un dispositif utilisateur, en particulier à un dispositif de consommateur portable, dans lequel le système comprend un moyen configuré pour mettre en œuvre le procédé selon l'une quelconque des revendications 1 à 13.

15. Produit-programme informatique comprenant un code de programme informatique capable d'amener un système informatique à mettre en œuvre un procédé tel que revendiqué dans l'une quelconque des revendications 1 à 13 lorsque le produit-programme informatique est exécuté sur un système informatique.
